# EUROPÄISCHE PATENTSCHRIFT

(11) **EP 3 466 107 B1**
(45) Veröffentlichungstag und Bekanntmachung des Hinweises auf die Patenterteilung: **05.08.2020**
(21) Anmeldenummer: 17731817.7
(22) Anmeldetag: 01.06.2017
(51) Int. Cl.: H04R 1/10

(54) **OHRSCHMUCK MIT INTEGRIERTEM HEADSET**
EAR JEWELLERY WITH INTEGRATED HEADSET
BOUCLE D'OREILLE POURVUE D'ÉCOUTEUR INTÉGRÉ

(30) Priorität: 03.06.2016 DE 102016110347
(43) Veröffentlichungstag der Anmeldung: 10.04.2019
(73) Patentinhaber: Nova Products GmbH, 80331 München (DE)
(72) Erfinder: GAMPE, Judith, 80636 München (DE)
(74) Vertreter: Hofstetter, Schurack & Partner
(86) Internationale Anmeldenummer: PCT/EP2017/063398
(87) Internationale Veröffentlichungsnummer: WO 2017/207736

(56) Entgegenhaltungen:
- EP-A1- 2 367 364
- CN-U- 204 741 538

## Beschreibung

Die Erfindung betrifft einen Ohrschmuck, der ein einzelnes Ohrstück oder zwei Ohrstücke umfassen kann, die in einer vorbestimmten Tragestellung (als Ohrclip, Ohrstecker oder Ohrring) am Ohr getragen werden können. Der Ohrschmuck weist des Weiteren elektrische Komponenten auf, um die Funktion eines kabellosen Headsets mit Mikrofon und Audio der der die Lautsprecher bereitzustellen.

Ein Headset ist eine Ergänzung zu einem portablen mobilen Endgerät, zum Beispiel ein Smartphone, oder eines Computers, um bei einem Telefongespräch über den Lautsprecher die Stimme des Gesprächspartners auszugeben und mittels des Mikrofons die Stimme des Benutzers des Endgeräts zu erfassen. Damit der Benutzer hierbei sein Endgerät nicht über ein Kabel mit dem am Kopf getragenen Headset verbinden muss, sondern frei zum Beispiel in einer Tasche eines Kleidungsstücks tragen kann, kann eine funkbasierte Kommunikationsverbindung zwischen dem Headset und dem Endgerät bereitgestellt werden.

Ein Headset ist in der Regel von anderen Personen erkennbar und stellt somit eine sichtbare Beeinflussung des äußeren Aussehens des Benutzers dar. Insbesondere weibliche Benutzer stören sich oftmals an auffälligen Gehäuseformen, die den technischen Charakter des Headsets sichtbar machen. Außerdem wird in den meisten Fällen der Lautsprecher direkt im Gehörgang platziert, was unkomfortabel für den Träger ist.

Aus der US 9,049,515, B2 ist ein Headset bekannt, dessen Elektronik hinter einer Ohrmuschel wie ein Hörgerät getragen werden kann. Der Lautsprecher des Headsets ist als einzige Komponente an der Vorderseite des Ohres angeordnet und durch eine Schmuckplatte kaschiert. Der Schall des Lautsprechers wird mittels eines Hörschlauchs in den Gehörgang des Benutzers geleitet. Aus dem Bereich hinter dem Ohr heraus für ein Telefonat den Sprachschall des Benutzers zu erfassen, ist ein stabförmiger Fortsatz vorgesehen, der von dem Bereich hinter dem Ohr zur Wange des Benutzers ragt. Aufgrund des durch den schallschlauch verschlossenen Gehörganges wirkt dieses Headset auf außenstehende Betrachter wie ein Hörgerät und veranlasst sie deshalb fälschlicherweise, besonders laut oder deutlich zu sprechen, was Träger des Headsets als unangenehm empfinden können. Zudem kann der stabförmiger Fortsatz des Mikrofons aufgrund seiner nicht offensichtlich erkennbaren Funktion auf Betrachter verwirrend wirken.

Aus der US 2008 0311966 A1 ist ein Headset bekannt, das wie ein Ohrring an der Ohrmuschel befestigt wird. Es besteht aus mehreren, auch optisch als verschieden zu identifizierbaren Komponenten. Die Schallübertragung erfolgt durch eine direkt im Gehörgang platzierte Komponente. Dies ist für den Anwender zum einen unbequem, da sich permanent ein Teil des Gerätes im Gehörgang befindet. Zum anderen wird dieses Teil optisch von umliegenden Personen wahrgenommen, und somit bleibt es als Headset identifizierbar. Eine Ausprägung schlägt vor, den Lautsprecher innerhalb der Ohrmuschel direkt vor dem Gehörgang zu platzieren. Hier muss dann teilweise Schall über den Knochen übertragen werden. Dennoch bleibt es als technisches Gerät identifizierbar und die Zierwirkung somit begrenzt.

Aus der EP 2 367 364 A1 ist ein drahtloses Audio-Headset bekannt, das aus zwei Komponenten besteht, nämlich einem Gehäuse und einer Ohr-Komponente, die im Falle der Nutzung des Headsets vom Gehäuse entfernt wird und am Ohr angebracht wird. Das Gehäuse beinhaltet zumindest einen Teil der technischen Schaltung, während sich zumindest der Lautsprecher in einer Gebrauchsstellung in der Ohr-Komponente befindet. Das Gehäuse befindet sich mit einer separaten Befestigung am Körper oder an der Kleidung der Person. Wird das Headset nicht verwendet, befindet sich auch die Ohrkomponente am Gehäuse. Wird es verwendet, so wird es vom Gehäuse entfernt, und am Ohr angebracht. Das Problem für den Nutzer besteht hier an dem umständlichen entfernen der Ohr-Komponente vom Gehäuse und anschließenden Wieder-Anfügen. Die integrierte Funktionalität ist somit nicht gegeben.

Aus EP 2 369 816 A1 ist ein System von Schmuckelementen bekannt, das ein entfernbares Kommunikationselement aufweist, welches einen großen Datenspeicher enthält und das man in einer mechanischen Halterung fixieren kann.

Dokument CN 204 741 538 U zeigt einen Ohrring mit einen integrierten Headset, wobei das Headset an einer Kette frei am Ohrläppchen pendeln kann.

Der Erfindung liegt die Aufgabe zugrunde, ein Headset bereitzustellen, das auch von Personen getragen werden kann, nicht wünschen, dass man ihnen die Benutzung des Headsets ansieht oder dass man den Schall aus dem Headset mithören kann.

Gleichzeitig soll das Headset nicht den Gehörgang blockieren und zusätzlich permanent am Ohr getragen werden können, damit es sofort verfügbar ist.

Die Aufgabe wird durch den Gegenstand von Patentanspruch 1 gelöst. Vorteilhafte Weiterbildungen der Erfindung sind durch die abhängigen Patentansprüche, die folgende Beschreibung sowie die Figuren offenbart.

Durch die Erfindung ist eine Ohrschmuckanordnung oder kurz ein Ohrschmuck bereitgestellt, der zumindest ein an jeweils einem Ohr eines Benutzers zu tragendes Ohrstück aufweist. Der Ohrschmuck kann also ein einzelnes Ohrstück oder zwei Ohrstücke vorsehen. Jedes Ohrstück kann jeweils z.B. als Ohrclip oder Ohrstecker oder Ohrring ausgestaltet sein. Jedes Ohrstück weist jeweils die folgenden beiden Elemente auf, nämlich einen Schmuckkörper und eine Halteeinrichtung. Der Schmuckkörper ist dazu eingerichtet, in einer vorbestimmten Tragestellung des Ohrstücks an einer vom Kopf des Benutzers abgewandten Seite des Ohrs getragen zu werden, d.h. an der Außenseite des Ohrs. Der Schmuckkörper ist somit in der Tragestellung von einer Umgebung aus sichtbar. Die Halteeinrichtung ist dazu eingerichtet, den Schmuckkörper in der besagten Tragestellung an einer Ohrmuschel des Ohres, insbesondere an einem Ohrläppchen oder an einer Helix des Ohres, zu tragen oder zu halten. In der Tragestellung bleibt somit der Gehörgang des Ohres stets frei. Insbesondere bleiben in der Tragestellung zusätzlich zum Gehörgang auch die Incisura Intertragica und der Cavum Conchae Inferor des Ohres frei. Jedes Ohrstück bedeckt also in der Tragestellung insbesondere nur das Ohrläppchen und/oder die Helix und/oder die Antihelix und/oder die Fossa Triangularis und/oder das Cavum Conchae superior. Die Tragestellung ist durch die Ausgestaltung der Halteeinrichtung und des Schmuckkörpers festgelegt. Insbesondere umfasst der Ohrschmuck ausschließlich das zumindest eine Ohrstück. Von dem Ohrschmuck ragt somit kein Element oder keine Komponente in den Gehörgang des Ohres hinein.

In dem zumindest einen Ohrstück sind insgesamt die folgenden elektrischen Komponenten vollständig und von außen unsichtbar integriert. Die Elemente können dabei in einem einzelnen Ohrstück angeordnet sein oder auf zwei Ohrstücke verteilt sein.

Eine erste Komponenten ist eine Mikrofoneinrichtung, also ein Schallwandler, der zum Erfassen eines von einem Mund des Kopfes des Benutzers zu dem Ohrstück gelangten Stimmschalls ausgelegt ist. Die Mikrofoneinrichtung kann dabei dazu eingerichtet sein, einen Luftschall zu erfassen, der vom Mund zu dem Ohrstück gelangt ist. Zusätzlich oder alternativ dazu kann die Mikrofoneinrichtung dazu eingerichtet sein, den Schall als Körperschall oder Knochenschall an einer Oberfläche des Kopfes und/oder des Ohres zu erfassen.

Des Weiteren ist als eine zweite Komponente eine Lautsprechereinrichtung zum Abstrahlen eines Lautsprecherschalls, d.h. eines Luftschalls, aus dem jeweiligen Ohrstück in Richtung des Gehörganges des jeweiligen Ohres vorgesehen. Die Lautsprechereinrichtung strahlt den Schall somit von dem Ohrstück ab, das von dem Gehörgang beabstandet angeordnet ist. Der Schall gelang dann durch die Luft der Umgebung in den Gehörgang. Hierbei hat sich herausgestellt, dass die Erzeugung des Schalls am Ohrläppchen oder an der Ohrmuschel des Ohres es ermöglicht, dass das Luftvolumen des Gehörganges selbst sich mittels der Lautsprechereinrichtung über diese Distanz hinweg derart in Schwingung versetzen lässt, dass eine natürlich klingende Übertragung des mittels des Schalls zu übertragenden Audiosignals ermöglicht ist. Vom Ohrläppchen oder der Ohrmuschel aus lässt sich nämlich die Luftsäule im Gehörgang des Ohrs über die Distanzen hinweg ausreichend zu einer Schwingung anregen, um ohne eine Anbindung über einen Schallschlauch den Schall an das Trommelfell des Benutzers zu führen.

Des Weiteren ist als eine dritte Komponente eine Kommunikationseinheit bereitgestellt, mit welcher die Mikrofoneinrichtung und die Lautsprechereinrichtung gekoppelt sein können. Die Kommunikationseinheit ist dazu eingerichtet, eine bidirektionale, kabellosen Kommunikationsverbindung zu einem portablen Endgerät des Benutzers bereitzustellen. Die Kommunikationsverbindung kann auf der Basis eines Bluetooth-Standards oder eines Wi-Fi-Standards oder auf einer Infrarot-Verbindung beruhen. Die Kommunikationseinheit ist dazu eingerichtet, über die Kommunikationsverbindung ein Mikrofonsignal der Mikrofoneinrichtung an das Endgerät auszusenden und aus dem Endgerät ein Lautsprechersignal für die Lautsprechereinrichtung zu empfangen. Somit kann der Ohrschmuck als Headset genutzt werden, um zum Beispiel ein Telefongespräch zu übertragen. Als Endgerät kann hierbei ein Telefon oder ein Smartphone oder ein Tablett-PC oder ein WLAN-Router (WLAN - Wireless Local Area Network) oder ein Kraftfahrzeug, wie zum Beispiel ein Personenkraftwagen, oder ein Personalcomputer vorgesehen sein.

Der Schmuckkörper kann zum Beispiel als Perle ausgestaltet sein und und/oder mit Edelsteinen oder Perlen besetzt sein und/oder eine Oberfläche mit einer Zierstruktur aufweisen. Ein Gehäuse des Schmuckkörpers kann insbesondere aus einem Edelmetall gebildet oder mit einem Edelmetall beschichtet sein. Als Form kann das Ohrstück insgesamt als Ohrring oder Ohrknopf oder Kreole oder Ohrstecker ausgestaltet sein. Die Erfindung versucht somit z.B. nicht, die Elektronik eines Headsets vollständig hinter dem Ohr eines Benutzers zu verstecken, sondern nutzt einen Innenraum eines Schmuckkörpers, der an einer Außenseite des Ohres getragen werden kann, um in seinem Innenraum eine oder einige oder alle der elektrischen Komponenten anzuordnen. Durch die Anordnung am Ohrläppchen oder an der Ohrmuschel und das Abstrahlen des Lautsprecherschalls in Richtung des Gehörganges lässt sich eine akustische Kopplung zwischen der schwingungsfähigen Luftsäule im Gehörgang und der Lautsprechereinrichtung erreichen, die eine Übertragung eines akustischen Signals (Stimme, Musik) als Lautsprecherschall ohne Hilfsmittel von dem Ohrstück bis zum Trommelfell ermöglicht.

Wie bereits ausgeführt, können die besagten elektrischen Elemente in dem Ohrschmuck auf zwei Ohrstücke verteilt angeordnet sein. Die Erfindung sieht aber vor, dass die Mikrofoneinrichtung und die Lautsprechereinrichtung in dem Schmuckkörper eines gemeinsamen Ohrstücks integriert sind und dieser Schmuckkörper ein Gehäuse aufweist, welches zumindest eine in der Tragestellung dem Gehörgang zugewandte Austrittsöffnung für den Lautsprecherschall und an einer gegenüberliegenden Seite eine Eintrittsöffnung für den Stimmschall aufweist. Mit anderen Worten weisen die zumindest eine Austrittsöffnung einerseits und die Eintrittsöffnung andererseits in entgegengesetzte Richtungen. Hierdurch ist eine Rückkopplung eines Stimmsignals eines Gesprächspartners des Benutzers unterdrückt oder zumindest verringert. Zusätzlich oder alternativ dazu kann vorgesehen sein, dass eine elektronische Wippe oder Wippschaltung vorgesehen ist, die zwischen dem Erfassen von Sprachschall einerseits und dem Ausgeben von Lautsprecherschall andererseits abwechselt wird, sodass im Betrieb nur entweder die Lautsprechereinrichtung oder die Mikrofoneinrichtung zur Zeit aktiv ist.

Wie bereits ausgeführt, ist es besonders wichtig, dass ausgehend von dem Ohrstück der Luftraum zwischen der Lautsprechereinrichtung und dem Gehörgang effektiv überwunden wird, sodass der Lautsprecherschall die Luftsäule im Gehörgang zu Schwingungen anregen kann. Die Lautsprechereinrichtung ist hierzu bevorzugt mit der Umgebung des Ohrstücks über eine Richteinrichtung gekoppelt. Die Richteinheit ist dazu eingerichtet, den Lautsprecherschall in der Tragestellung in eine Richtung zu dem Gehörgang hin auszurichten, sodass der Lautsprecherschall nach dem Austritt aus dem Ohrstück in der Umgebung eine Hauptausbreitungsrichtung aufweist, d.h. eine Richtung der größten Lautstärke, entlang welcher der Lautsprecherschall in den Gehörgang gelangt oder diesen zumindest passiert. Mit anderen Worten wird mittels der Richteinheit ein gerichteter Schall von der Lautsprechereinrichtung aus dem Ohrstück abgestrahlt. Falls die Hauptausbreitungsrichtung des Luftschalls durch die Richteinheit an dem Gehörgang lediglich vorbeigeführt wird, so ergibt sich aufgrund von Beugung im Bereich des Gehörgangs, d.h. dessen Eingangsöffnung, eine ausreichend große akustische Kopplung, um dennoch die Luftsäule im Gehörgang zu Schwingungen anzuregen. Der gerichtete Lautsprecherschall weist den zusätzlichen Vorteil auf, dass eine andere Person, die sich in der Nähe des Benutzers befindet, den Lautsprecherschall nicht ebenfalls so deutlich hören kann, dass sie zum Beispiel den Inhalt eines Gesprächs mithören könnte.

Zu der Erfindung gehören auch vorteilhafte Weiterbildungen, durch deren Merkmale sich zusätzliche Vorteile ergeben.

Mehrere Austrittsöffnungen können z.B. durch einen Bereich eines Gehäuses des Schmuckkörpers gebildet sein, der vergittert ist oder mehrfach perforiert oder durchlöchert ist, d.h. eine Anordnung mehrerer Durchgangsöffnungen oder Löcher aufweist, die in insgesamt für Frequenzen des Lautsprecherschalls passierbar oder durchdringbar sind.

Eine Weiterbildung sieht vor, dass für eine Rückkopplungsunterdrückung eines Schallanteils des Lautsprecherschalls, der aus der Lautsprechereinrichtung kommt und sich in Richtung zur Mikrofoneinrichtung ausbreitet eine Prozessoreinheit des Ohrschmucks dazu eingerichtet ist, ein von der Mikrofoneinrichtung erfasstes Schallsignal des Schallanteils zu dämpfen. Die Prozessoreinheit kann hierzu einen Algorithmus der Echokompensation durchführen. Zusätzlich oder alternativ ist für eine Rückkopplungsunterdrückung bevorzugt vorgesehen, dass das Gehäuse zwei Kammern aufweist und die Mikrofoneinrichtung und die Lautsprechereinrichtung in unterschiedlichen der Kammern angeordnet sind. Die Mikrofoneinrichtung und die Lautsprechereinrichtung sind somit durch eine Wandung der Kammern getrennt. Hierdurch ist auch ein Übersprechen von der Lautsprechereinrichtung in die Mikrofoneinrichtung vermieden, da der in dem Ohrstück erzeugte Lautsprecherschall sich innerhalb des Ohrstücks nicht bis zur Mikrofoneinrichtung ausbreiten kann.

Als Richteinheit kann zum Beispiel ein in dem Ohrstück angeordneten Trichter vorgesehen sein, welcher die Hauptausbreitungsrichtung des Lautsprecherschalls in der beschriebenen Weise ausrichtet. Zusätzlich oder alternativ dazu kann ein in dem Ohrstück angeordneter Schlauch oder ein Rohr vorgesehen sein, welches den Lautsprecherschall von der Lautsprechereinrichtung hin zu einer Austrittsöffnung führt und hierbei die Hauptausbreitungsrichtung in der beschriebenen Weise ausrichtet. Zusätzlich oder alternativ dazu können mehrere in einer Außenwandung des Ohrstücks angeordnete Durchgangsöffnungen in der beschriebenen Weise vorgesehen sein, d.h. mehrere Austrittsöffnungen. Die Durchgangsöffnungen sind dabei in der Weise relativ zueinander angeordnet, dass der jeweilige aus der Durchgangsöffnungen austretende Luftschall sich außerhalb des Ohrstücks mit dem Schall aus den übrigen Durchgangsöffnungen akustisch überlagert und sich hierdurch ein sogenanntes Beamforming ergibt, durch welches die Hauptausbreitungsrichtung des Lautsprecherschalls ausgerichtet wird.

Durch ein Rohr kann auch eine Resonanz für zumindest eine Frequenz des Lautsprecherschalls bewirkt werden und hierdurch der Austritt einer Energie oder einer Leistung des Lautsprecherschalls aus einer dem Rohr nachgeordneten Austrittsöffnung begünstigt werden.

Eine Weiterbildung sieht vor, dass der Ohrschmuck eine Lautstärkeregulierung zum Einstellen einer Lautstärke des Lautsprecherschalls und/oder einer Verstärkung des Mikrofonsignals in Abhängigkeit von zumindest einem Bediensignal aufweist, wobei das zumindest eine Bedienelement zumindest eines der folgenden umfasst. Es kann zumindest ein Sensor vorgesehen sein, der ein Bediensignal in Abhängigkeit von einer Erschütterung des zumindest eines Ohrstücks erzeugt. Hierdurch kann die Lautstärke durch Antippen des zumindest einen Ohrstücks eingestellt werden. Ein geeigneter Sensor ist ein Beschleunigungssensor. Es kann eine Analyseeinrichtung vorgesehen sein, die in dem Mikrofonsignal der Mikrofoneinrichtung zumindest einen vorbestimmten Impuls detektiert und daraufhin ein Bediensignal erzeugt. Somit kann ein Klapse am Gehäuse registriert und zum Einstellen der Lautstärke genutzt werden. Die Analyseeinrichtung kann in bekannter Weise als eine Software der besagten Prozessoreinheit oder als ein integrierter Schaltkreis ausgestaltet sein. Es kann vorgesehen sein, zumindest einen Sensor bereitzustellen, der ein Bediensignal in Abhängigkeit von einer Streichbewegung eines Objekts, zum Beispiel eines Fingers, an einer Außenoberfläche des zumindest einen Ohrstücks erzeugt. Es kann hierzu ein einzelner kapazitiver Näherungssensor dazu vorgesehen sein, während der Streichbewegung des Objekts ein Näherungssignal mit einem zeitlichen Verlauf zu erzeugen, der ein Vergrößern oder Verkleinern der Lautstärke signalisiert. Zusätzlich oder alternativ dazu kann eine Reihe oder ein Array aus mehreren Näherungssensoren vorgesehen sein, die in einer vorbestimmten Reihenfolge während der Streichbewegung nacheinander eine Näherung oder ein Entfernen signalisieren, wodurch ebenfalls zwischen einem Vergrößern und einem Verkleinern Lautstärke unterschieden werden kann. Es können also Sensoren derartig integriert sein, dass durch "Darüberstreichen" eine Lautstärkeregulierung ermöglicht ist. Es kann ein Drehlagesensor vorgesehen sein, der bei Drehen eines Teilstücks des Ohrschmucks ein Bediensignal in Abhängigkeit von einer absoluten Drehlage im Raum oder in Abhängigkeit von einer relativen Drehlage des Teilstück bezüglich eines Rests des Ohrschmucks erzeugt. Der Drehlagesensor kann als Drehsteller ausgestaltet sein oder aber auch die Drehlage eines Bauteils des Ohrschmuck bezüglich des Rest erfassen, z.B. die Drehlage des Schmuckkörpers absolut im Raum oder bezüglich der Halteeinrichtung.

Eine Weiterbildung sieht vor, dass eine Energiespeichereinrichtung zum Betreiben der elektrischen Komponenten in den Ohrschmuck derart integriert ist, dass die Energiespeichereinrichtung in der Tragestellung hinter der Ohrmuschel zwischen Ohrmuschel und Kopf des Benutzers oder unter der Ohrmuschel angeordnet ist, wobei die Energiespeichereinrichtung über zumindest ein elektrisches Leitelement elektrisch mit den elektrischen Komponenten verbunden ist und das zumindest eine Bedienelement dazu eingerichtet ist, das zumindest eine Bediensignal auf eine in dem zumindest einen elektrischen Leitelement bereitgestellte elektrische Spannung aufzumodulieren. Allgemein kann vorgesehen sein, dass eine Modulationseinrichtung dazu eingerichtet ist, zumindest ein Sensorsignal und/oder zumindest ein Signal eines Bedienelements auf eine in dem zumindest einen elektrischen Leitelement bereitgestellte elektrische Spannung aufzumodulieren. Somit ist keine zusätzliche Verkabelung zum Verbinden der Bedienelemente mit im Schmuckköper angeordneten elektrischen Komponenten nötig.

Wie bereits beschrieben wird jeder Schmuckkörper des Ohrschmucks durch eine jeweilige Halteeinrichtung an dem Ohr gehalten. Als Halteeinrichtung kann zum Beispiel eine Klammer oder ein Clip vorgesehen sein, um den Schmuckkörper durch Klemmen an der Ohrmuschel oder dem Ohrläppchen zu halten. Ein solcher Clip kann zum Beispiel auf der Grundlage einer Blattfeder oder Schenkelfeder gebildet sein. Die Halteeinrichtung kann auch einen sogenannten Ohrstecker vorsehen, der durch ein sogenanntes Ohrloch, d.h. einen Durchstich im Ohrläppchen oder der Ohrmuschel, gesteckt werden kann. Der Ohrstecker weist insbesondere einen Durchmesser kleiner als 4 mm auf. Im Ohrloch ist entweder keine elektrische Komponente oder nur eine elektrische Durchleitung vorgesehen, aber keine elektronische Schaltungskomponente. Bevorzugt ist vorgesehen, dass die Energiespeichereinrichtung zum Betreiben der besagten elektrischen Komponenten, d.h. der Kommunikationseinheit, der Lautsprechereinrichtung und der Mikrofoneinrichtung, in die Halteeinrichtung integriert ist. Es ergibt sich der Vorteil, dass eine verhältnismäßig schwere Komponente des Ohrschmucks, nämlich die Energiespeichereinrichtung, nahe am Ohr getragen wird. Hierdurch kann zum Beispiel ein unangenehmes Gefühl am Ohr verhindert werden. Zudem ergibt sich ein eine Balance der Bestandteile des Ohrschmucks bezüglich des Ohrs, sodass der Ohrschmuck keine unerwünschte Schräglage aufweist. Als Energiespeichereinrichtung kann zum Beispiel eine Batterie vorgesehen sein, insbesondere eine Knopfzelle.

Für den Fall, dass die Halteeinrichtung einen Ohrstecker aufweist, also zum Beispiel einen Stab oder ein Rohr, der zum Anordnen in einer Perforierung (Ohrloch) des Ohres vorgesehen ist, ist bevorzugt als Bestandteil der Halteeinrichtung auch ein in der Tragestellung an einer dem Schmuckkörper gegenüberliegenden Seite des Ohres angeordnetes Abschlusselement vorgesehen, das an dem Ohrstecker zu befestigen ist. Die dem Schmuckkörper gegenüberliegenden Seite des Ohres ist die Rückseite des Ohres, also der Bereich zwischen dem Ohr und den Kopf. Dort ist für den Ohrstecker das Abschlusselement vorzusehen, damit der Stab oder das Rohr, das heißt der Ohrstecker, nicht aus der Perforierung herausrutschen kann. Bevorzugt ist nun vorgesehen, dass die besagte Energiespeichereinrichtung in dem Abschlusselement angeordnet ist. In dem Ohrstecker selbst ist entsprechend ein elektrischer Draht oder ein Metallstift angeordnet, welcher dazu eingerichtet ist, die Energiespeichereinrichtung elektrisch mit dem Schmuckkörper zu verbinden. Der Ohrstecker kann also ein Rohr sein mit einem innen liegenden Metalldraht oder Metallstift. Der Metalldraht beziehungsweise der Metallstift ist dabei elektrisch gegenüber der Außenwandung des Ohrsteckers isoliert. Es kann vorgesehen sein, dass mittels einer Feder ein elektrischer Kontakt an einem Ende des Ohrsteckers federnd gegen die Energiespeichereinrichtung drückt. Hierdurch ist auch bei einer Bewegung des Ohres eine zuverlässige Übertragung von elektrischer Energie gewährleistet.

Für den Fall, dass die Energiespeichereinrichtung auf der Rückseite des Ohres und der Schmuckkörper an der Vorderseite des Ohres angeordnet ist, ist bevorzugt vorgesehen, dass ein Gewicht des Abschlusselements (mit der darin angeordneten Energiespeichereinrichtung) einerseits und ein Gewicht des Schmuckkörpers andererseits sich um höchstens den Faktor 2, insbesondere höchstens den Faktor 1,5, unterscheiden. Hierdurch ergibt sich eine Verteilung des Gewichts vor und hinter dem Ohrläppchen bzw. der Ohrmuschel, sodass der Ohrschmuck nicht die besagte, unerwünschte Schräglage einnimmt.

Eine Weiterbildung sieh vor, dass zumindest ein Sensor in dem Ohrschmuck bereitgestellt ist, der dazu ausgelegt ist, in der Tragestellung zumindest eine vorbestimmte Bewegung des Kopfes im Raum zu erfassen und dann ein vorbestimmtes Steuersignal für die elektrischen Komponenten zu erzeugen. Als Sensor kann Beschleunigungssensor vorgesehen sein. Somit ist es möglich, bei einem Nicken des Kopfes ein Telefongespräch anzunehmen und bei einem Kopfschütteln des Kopfes das Telefongespräch abzulehnen.

Um die elektrischen Komponenten energiesparend betreiben zu können, ist bevorzugt vorgesehen, dass eine Steuerschaltung des Ohrschmucks dazu eingerichtet ist, in einem Ruhezustand die Lautsprechereinrichtung und die Mikrofoneinrichtung stromlos zu halten, also nicht zu betreiben oder abgeschaltet zu halten. Hierdurch kann auch eine elektromagnetische Strahlung, die von einer elektrischen Schaltung der Lautsprechereinrichtung und/oder Mikrofoneinrichtung ausgehen kann, reduziert werden. Die Steuerschaltung kann des Weiteren dazu eingerichtet sein, in Abhängigkeit von einem kabellos empfangenen Aufwecksignal des Endgeräts die Lautsprechereinrichtung und die Mikrofoneinrichtung mit Strom zu versorgen. Mit anderen Worten werden also eingehende Telefongespräche sofort angenommen, indem mittels des Aufwecksignals die Lautsprechereinrichtung und die Mikrofoneinrichtung eingeschaltet werden. Damit kann der Ohrschmuck bedienelementlos ausgeschaltet werden.

Alternativ dazu kann ein Bedienelement vorgesehen sein, welches zu betätigen ist, um zum Beispiel ein eintreffendes Telefonat anzunehmen, wobei es hier dazu vorgesehen wäre, die Lautsprechereinrichtung und/oder die Mikrofoneinrichtung und/oder die Kommunikationseinheit einzuschalten. Ein solches Bedienelement kann z.B. eine mechanische Taste und/oder einen Näherungssensor zum berührungslosen Schalten aufweisen. Das Vorsehen eines Bedienelements weist den Vorteil auf, dass eine Sprechverbindung zu einem Gesprächspartner nicht unkontrolliert aufgebaut wird. Zusätzlich oder alternativ dazu kann das Bedienelement dazu eingerichtet sein, in Abhängigkeit von einer Benutzerbetätigung eine Lautstärke der Lautsprechereinrichtung und/oder der Mikrofoneinrichtung zu regulieren oder einzustellen oder festzulegen. Das Bedienelement kann hierzu ein Drehrad aufweisen.

Eine Weiterbildung sieht vor, dass eine Steuerschaltung des Ohrstücks dazu eingerichtet ist, in einem Ruhezustand die Lautsprechereinrichtung und/oder die Mikrofoneinrichtung und/oder die Kommunikationseinheit stromlos zu halten und sie in Abhängigkeit von einer vorbestimmten Bedienhandlung des Benutzers mit elektrischem Strom zu versorgen und bevorzugt auch erst in Abhängigkeit von der Bedienhandlung die Kommunikationsverbindung aufzubauen. Die Bedienhandlung kann mit dem zumindest einen besagten Bedienelement erfasst werden. Das zumindest eine Bedienelement kann somit zusätzlich oder alternativ für die Lautstärkeregulierung und/oder die Aktivierung vorgesehen sein.

Wenn also gerade kein Telefonanruf getätigt oder empfangen wird, kann die Kommunikationseinheit abgeschaltet sein. Es kann hierzu vorgesehen sein, dass sich die Kommunikationseinheit selbständig abschaltet, falls über die Kommunikationsverbindung zum Endgerät für eine vorbestimmte Mindestzeitdauer kein Signal übertragen wird. Zusätzlich oder alternativ dazu kann ein Bedienelement zum Abschalten der Kommunikationseinheit vorgesehen sein, beispielsweise eine Taste oder ein anderes der bereits beschriebenen Bedienelemente.

Falls dann ein Telefonanruf eintrifft, kann er von dem Endgerät empfangen werden. Das Endgerät kann dann ein Signal (akustisch und/oder mittels Vibration) ausgeben. Der Benutzer kann dann den Ohrschmuck mittels des besagten Bedienelements wieder einschalten, d.h. die Kommunikationseinheit aktivieren, woraufhin von der Kommunikationseinheit die Kommunikationsverbindung zum Endgerät aufgebaut wird und der Telefonanruf über den Ohrschmuck angenommen werden kann, ohne dass das Endgerät selbst bedient werden muss. Der Verbindungsaufbau ist ausreichend schnell, sodass das Endgerät für die rechtzeitige Rufannahme nicht bedient oder in die Hand genommen werden muss.

Der Ohrschmuck kann somit ausgeschaltet bleiben, solange kein Anruf getätigt wird. Entscheidender Vorteil ist hierbei, dass die Strahlungsbelastung durch den Ohrschmuck für Benutzer minimiert wird, da die Kommunikationsverbindung und die damit einhergehende elektromagnetische Strahlung minimiert werden.

Falls der Benutzer selbst einen Anruf tätigen möchte, kann er die Kommunikationseinheit ebenfalls mittels des besagten Bedienelements einschalten und z.B. mittels Sprachbedienung das Anwählen einer Telefonnummer im Endgerät auslösen, ohne dieses zur nehmen und manuell bedienen zu müssen.

Ein weiterer Vorteil ergibt sich, wenn jeder Schmuckkörper jeweils an einer in der Tragestellung der Umgebung zugewandten Seite ein von außen sichtbares, austauschbares Zierelement aufweist, welches von einem Rest des Schmuckkörper zerstörungsfrei und reversibel lösbar ausgestaltet ist. Dies weist Vorteil auf, dass sich das von außen sichtbare Aussehen des Schmuckkörpers verändern lässt, ohne dass hierzu auch die elektrischen Komponenten neu gekauft oder beschafft werden müssen. Das Zierelement kann beispielsweise an den Rest des Schmuckkörpers geklipst oder geschraubt sein. Das Zierelement kann ein Gehäuse des Schmuckkörpers sein, in welchem die Mikrofoneinrichtung und die Lautsprechereinrichtung angeordnet sein können.

Eine Weiterbildung sieht vor, dass der Schmuckkörper mindestens eine Halterung oder ein Halteelement zum wahlweise Befestigen und/oder Anhängen zumindest eines austauschbaren Zusatzzierelements aufweist. Das Halteelement kann als Öse oder Haken ausgestaltet sein.

Eine Weiterbildung sieht vor, dass ein Vibrationselement bereitgestellt ist, welches dazu eingerichtet ist, in Abhängigkeit von einem Aktivierungssignal des Endgeräts bei Eintreffen einer Nachricht oder Anrufes ein Vibrationssignal zu erzeugen und/oder bei Andrücken des Ohrstücks, in welchem das Vibrationselement angeordnet ist, an den Kopf das Lautsprechersignal als einen Körperschall in den Kopf des Benutzers abzugeben. Das andrücken kann mittels eines Sensors erkannt oder sensiert werden. Beispielsweise kann hierzu ein Mikrotaster vorgesehen sein. Durch diese Weiterbildung ist eine Schallübertragung oder Schallabnahme über den Knochen des Schädels des Kopfes geleitet.

Eine Weiterbildung sieht vor, dass der Ohrschmuck zumindest einen Sensor zur Erfassung von Körperdaten, insbesondere Herzfrequenz und Temperatur, aufweist. Hierdurch kann z.B. eine sportliche Performanz des Benutzers abgeleitet werden.

Eine Weiterbildung sieht vor, dass der Ohrschmuck über ein Panik-Bedienelement verfügt, das bei Betätigung einen Notruf in dem Endgerät auslöst.

Eine Weiterbildung sieht vor, dass der Ohrschmuck über eine Haltefunktion verfügt, die dazu eingerichtet ist, in Abhängigkeit von einer vorbestimmten ersten Bediengeste eine eingehende Sprechverbindung zu halten und in Abhängigkeit von einer vorbestimmten zweiten Bediengeste die Sprechverbindung wieder durchzuschalten oder wieder anzunehmen.

Im Folgenden ist ein Ausführungsbeispiel der Erfindung beschrieben. Hierzu zeigt:
- Fig. 1: eine schematische Darstellung einer perspektivischen Ansicht eines Benutzers mit einer Ausführungsform des erfindungsgemäßen Ohrschmucks und mit einem mobilen Endgerät;
- Fig. 2: eine schematische Darstellung einer Frontalansicht eines Ohres des Benutzers mit dem Ohrschmuck von Fig. 1;
- Fig. 3: eine schematische Darstellung einer Schnittansicht eines Schmuckkörpers des Ohrschmucks von Fig. 1;
- Fig. 4: eine schematische Darstellung einer Schnittansicht eines Abschlusselements der Halteeinrichtung des Ohrstücks von Fig. 1;
- Fig. 5: eine schematische Darstellung einer Schnittansicht eines Ohrsteckers aus einer Halteeinrichtung des Ohrschmucks von Fig. 1;
- Fig. 6: eine schematische Darstellung einer Ohrmuschel zur Veranschaulichung der in dieser Beschreibung verwendeten Begriffe von Bestandteilen der Ohrmuschel;
- Fig. 7: eine schematische Darstellung einer weiteren Ausführungsform des erfindungsgemäßen Ohrschmucks;
- Fig. 8: eine schematische Darstellung einer weiteren Ausführungsform des erfindungsgemäßen Ohrschmucks mit einer Signalmodulation einer Versorgungsspannung;
- Fig. 9: eine schematische Darstellung einer weiteren Ausführungsform des erfindungsgemäßen Ohrschmucks mit einem austauschbaren Zierelement;
- Fig. 10: eine schematische Darstellung einer weiteren Ausführungsform des erfindungsgemäßen Ohrschmucks mit einem Halteelement zum Befestigen und/oder Anhängen eines Zusatzzierelements; und
- Fig. 11: eine schematische Darstellung einer weiteren Ausführungsform des erfindungsgemäßen Ohrschmucks, bei welchem eine Energiespeichereinrichtung in dem Schmuckkörper integriert ist; und
- Fig. 12: eine schematische Darstellung einer weiteren Ausführungsform des erfindungsgemäßen Ohrschmucks, bei welcher eine Kommunikationseinheit in eine Halteeinrichtung des Ohrschmucks integriert ist; und
- Fig. 13: eine schematische Darstellung einer weiteren Ausführungsform des erfindungsgemäßen Ohrschmucks, bei welcher die Kommunikationseinheit in die Halteeinrichtung des Ohrschmucks integriert ist.

Bei dem im Folgenden erläuterten Ausführungsbeispiel handelt es sich um eine bevorzugte Ausführungsform der Erfindung. Bei dem Ausführungsbeispiel stellen die beschriebenen Komponenten der Ausführungsform jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren ist die beschriebene Ausführungsform auch durch weitere der bereits beschriebenen Merkmale der Erfindung ergänzbar.

In den Figuren sind funktionsgleiche Elemente jeweils mit denselben Bezugszeichen versehen.

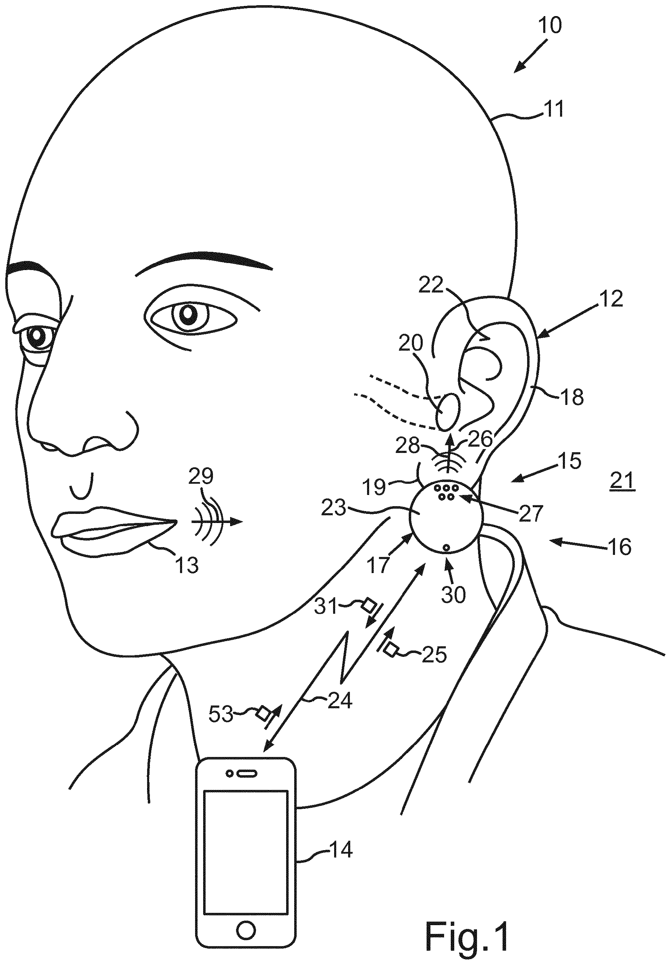

Fig. 1 zeigt von einem Benutzer 10 dessen Kopf 11 mit einem Ohr 12 und einem Mund 13. Der Benutzer 10 kann ein portables mobiles Endgerät 14 benutzen, um zum Beispiel eine Telefonverbindung mit einem Gesprächspartner (nicht dargestellt) zu empfangen oder aufzubauen. Bei dem mobilen Endgerät 14 kann es sich zum Beispiel um ein Smartphone oder ein anderes der bereits genannten Geräte handeln. Zum Telefonieren muss der Benutzer 10 das Endgerät 14 nicht an sein Ohr 12 halten. Stattdessen kann der Benutzer 10 ein Headset 15 benutzen. Dieses Headset 15 ist durch einen Ohrschmuck 16 bereitgestellt, der in dem in Fig. 1 veranschaulichten Beispiel ein Ohrstück 17 aufweist, dass an einer Ohrmuschel 18 oder (in Fig. 1 dargestellt) an einem Ohrläppchen 19 des Ohres 12 befestigt sein kann. Im befestigten Zustand weist das Ohrstück 17 eine Tragestellung T auf. Ein Gehörgang 20 des Ohrs 12 ist in der Tragestellung T freigelassen oder frei zugänglich, d.h. nicht von dem Ohrschmuck 16 bedeckt, sodass dem Ohrschmuck 16 nicht angesehen werden kann, dass es sich um ein Headset 15 handelt. Die Tragestellung T des Ohrstücks 17 kann in bekannter Weise durch Ausgestalten des Schmuckkörpers 23 und der Halteeinrichtung 32 erreicht werden. Zusätzlich kann eine die Tragestellung T veranschaulichende Bedienungsanleitung bereitgestellt sein.

Von einer Umgebung 21 aus, d.h. von einer Außenseite 22 des Ohrs 12 aus, ist von dem Ohrstück 12 ein Schmuckkörper 23 wie bei jedem bekanntem Ohrschmuck zu sehen. In dem in Fig. 1 veranschaulichten Beispiel ist der Schmuckkörper 23 als eine Perle oder Kugel ausgestaltet. Es kann auch eine andere der genannten Formen für das Ohrstück 17 vorgesehen sein.

Für das Telefonat kann eine kabellose Kommunikationsverbindung 24 zwischen dem Endgerät 14 und dem Ohrstück 17 aufgebaut werden. Über die Kommunikationsverbindung 24 an ein Lautsprechersignal 25 zu dem Ohrstück 17 übertragen werden, damit das Ohrstück 17 aus dem Lautsprechersignal 25 einen Lautsprecherschall 26 erzeugt, der aus mehreren Austrittsöffnungen 27 des Schmuckkörpers 23 in die Umgebung 21 austreten und mit einer Hauptausbreitungsrichtung 28 auf den Gehörgang 20 ausgerichtet sein kann. Ein Sprachschall 29 aus dem Mund 13 des Benutzers 10 kann durch eine Eintrittsöffnung 30 des Ohrstücks 17 in das Ohrstück 17 gelangen und dort in ein Mikrofonsignal 31 umgewandelt werden, welches über die Kommunikationsverbindung 24 zu dem Endgerät 14 übertragen werden kann.

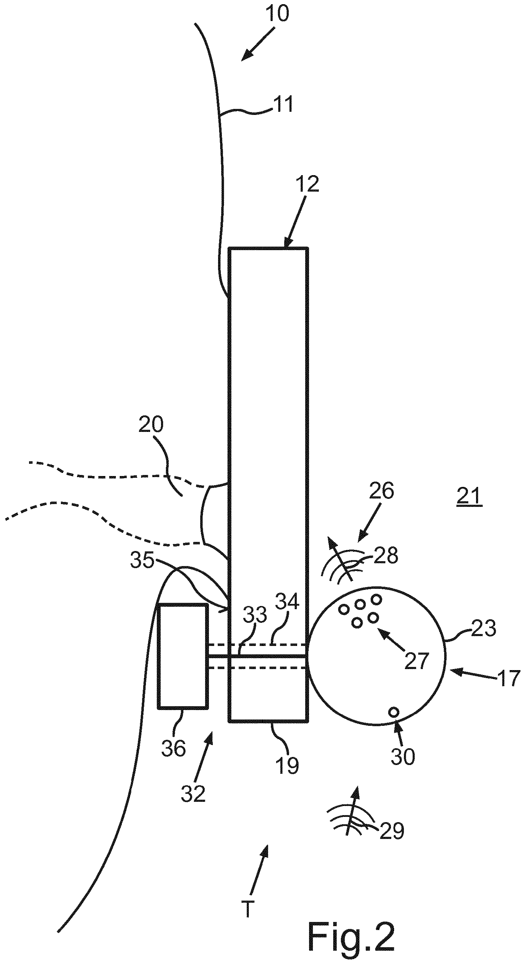

Fig. 2 veranschaulicht, wie der Schmuckkörper 23 durch eine Halteeinrichtung 32 an zum Beispiel dem Ohrläppchen 19 gehalten werden kann. Die Halteeinrichtung 32 kann einen Ohrstecker 33 aufweisen, der durch ein Ohrloch 34 des Ohrläppchens 19 gesteckt sein kann. Der Ohrstecker weist insbesondere einen Durchmesser kleiner als 4 mm auf. Damit kann auch ein gewöhnliches Schmuck-Ohrloch 34 genutzt werden, d.h. es ist kein gedehntes Piercing notwendig. Auf einer der Umgebung 21 abgewandten Seite oder Rückseite 35 des Ohrs 12 kann die Halteeinrichtung 32 ein Abschlusselement 36 aufweisen, welches ein Herausrutschen des Ohrsteckers 33 aus der Perforierung 34 blockiert.

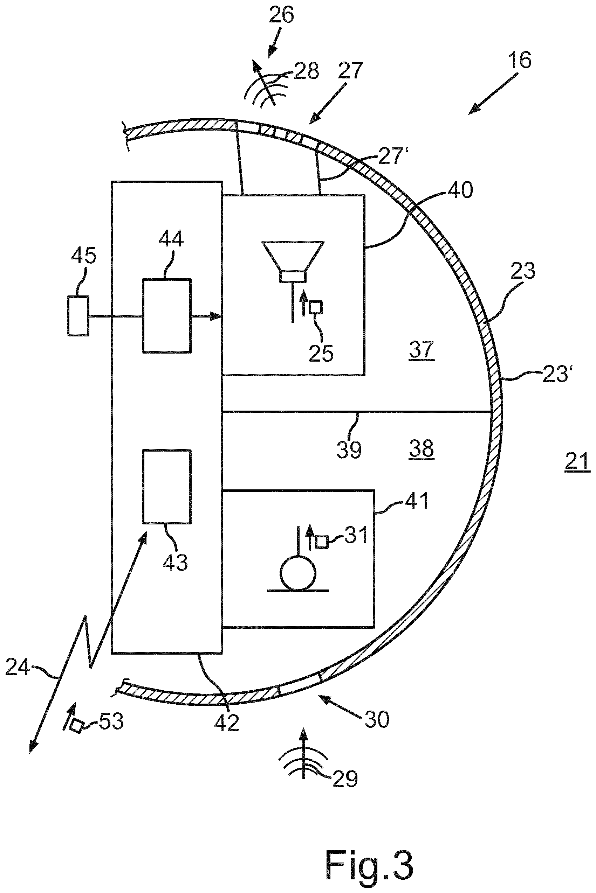

Fig. 3 veranschaulicht eine mögliche Ausgestaltung des Schmuckkörpers 23. Ein Gehäuse 23' des Schmuckkörpers 23 kann zwei Kammern 37, 38 aufweisen, die durch eine Kammerwand 39 voneinander getrennt sein können, so das ein Übertritt von Schall zwischen den Kammern 37, 38 gedämpft ist. In der Kammer 37 kann eine Lautsprechereinrichtung 40 angeordnet sein, durch welche ein Lautsprecher bereitgestellt sein kann, welcher das Lautsprechersignal 25 in den Lautsprecherschall 26 wandelt. In der Kammer 38 kann eine Mikrofoneinrichtung 41 bereitgestellt sein, durch welche mittels eines Mikrofons der Sprachschall 29 in das Mikrofonsignal 31 gewandelt werden kann. Die Kammer 37 ist über die Austrittsöffnungen 27 mit der Umgebung 21 fluidisch gekoppelt. Die Kammer 38 ist über die Eintrittsöffnung 30 mit der Umgebung 21 fluidisch gekoppelt.

Eine Anordnung der Austrittsöffnungen 27 für den Lautsprecherschall 26 kann derart gewählt sein, dann sich den aus den einzelnen Austrittsöffnungen 27 austretenden Schall aufgrund eines Überlagerungseffekts in der Umgebung 21 eine Richtwirkung oder ein Beamforming ergibt, durch welches die Hauptabstrahlrichtung 28 zu dem Gehörgang 20 ausgerichtet wird. Die Anordnung kann z.B. mittels eine Simulationsprogramms ermittelt werden. Dies kann zusätzlich oder alternativ durch ein Rohr 27' oder einen Schlauch oder eine Trichter bewirkt werden, über welchen die Lautsprechereinrichtung 40 mit den Austrittsöffnungen 27 gekoppelt sein kann. Die derart gestalteten Austrittsöffnungen 27 und/oder der Schlauch 27' stellen eine Richteinheit dar.

Zum Betreiben der Lautsprechereinrichtung 40 und der Mikrofoneinrichtung 41 kann eine Schaltungsplatine 42 bereitgestellt sein. Die Schaltungsplatine 42 kann als weitere Komponenten eine Kommunikationseinheit 43 aufweisen, bei der es sich z.B. um ein Bluetooth-Funkmodul handeln kann. Mittels der Kommunikationseinheit 43 kann die Kommunikationsverbindung 24 mit dem Endgerät 14 aufgebaut werden.

Des Weiteren kann eine Steuerschaltung 44 bereitgestellt sein, mittels welcher die Lautsprechereinrichtung 40 und die Mikrofoneinrichtung 41 betrieben werden können. Es kann zum Beispiel ein Bedienelement 45 vorgesehen sein, mittels welchem eine Lautstärke der Lautsprechereinrichtung 40 und oder der Mikrofoneinrichtung 41 durch Einstellen einer Verstärkung festgelegt werden kann. Das Bedienelement 45 kann zum Beispiel in dem Abschlusselement 36 bereitgestellt sein (siehe Fig. 2). Durch die Steuerschaltung 44 können die Lautsprechereinrichtung 40 und/oder die Mikrofoneinrichtung 41 auch ein geschaltet und ausgeschaltet werden. Das Bedienelement 45 kann hierzu ebenfalls eine Bedienmöglichkeit aufweisen, z.B. einen Taster oder einen Näherungssensor.

Bei dem Schmuckkörper 23 kann das Gehäuse 23' Zierelement abnehmbar oder austauschbar ausgestaltet sein. So kann eine aus der Umgebung 21 sichtbare Zieroberfläche des Schmuckkörpers 23 unter Beibehaltung der elektrischen Komponenten ausgewechselt werden. Das Gehäuse 23' stellt somit ein Zierelement dar.

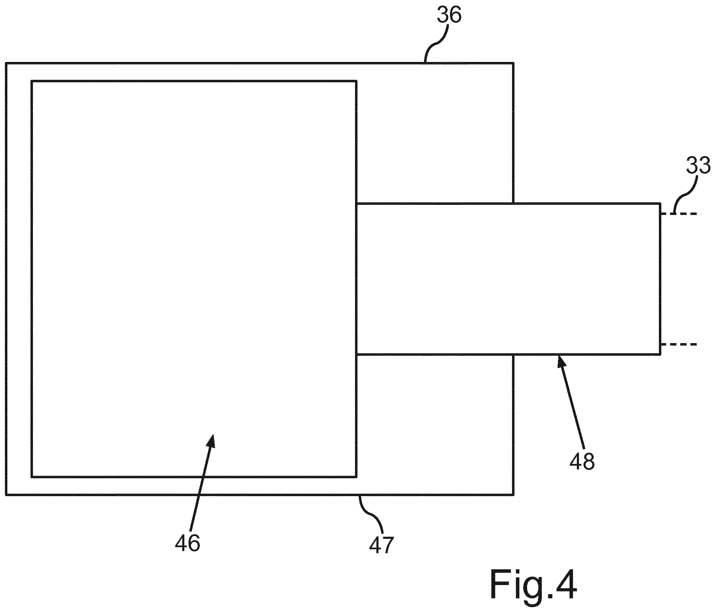

Fig. 4 veranschaulicht das Abschlusselement 36, in welchem eine Energiespeichereinrichtung 46 angeordnet sein kann, zum Beispiel eine Batterie, insbesondere eine Knopfzelle. Die Energiespeichereinrichtung 46 kann in einem Gehäuse 47 des Abschlusselements 36 angeordnet sein. An dem Ohrstecker 33 kann das Abschlusselement 36 durch ein Verbindungselement 48 gehalten sein, bei dem es sich zum Beispiel um einen Rohrstutzen handeln kann, dass auf den Ohrstecker 33 aufgesteckt ist.

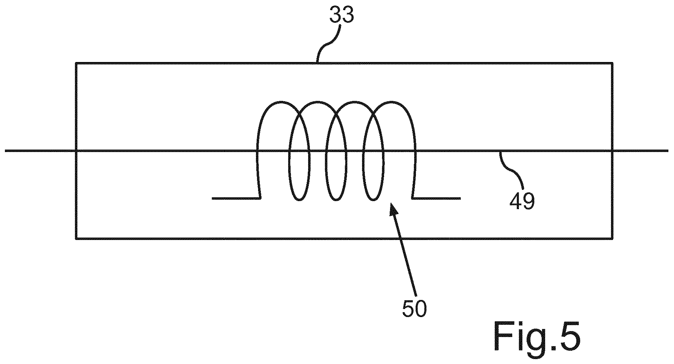

Fig. 5 veranschaulicht den Ohrstecker 33, der beispielsweise als ein Rohr ausgestaltet sein kann. Der Ohrstecker stellt einen Verbindungsstecker zwischen dem Schmuckkörper 23 und dem Abschlusselement 36 dar.

In dem Ohrstecker 33 kann ein Metalldraht oder ein Metallstift 49 angeordnet sein, über welchen ein elektrischer Strom von der Energiespeichereinrichtung 46 zu der Lautsprechereinrichtung 40 und der Mikrofonrichtung 41 übertragen werden kann. Mit anderen Worten kann insbesondere die Schaltungsplatine 42 mit elektrischer Energie aus der Energiespeicherrettung 46 über den Metallstift 49 versorgt werden. Mittels einer Feder 50, die in dem Ohrstecker 33 angeordnet ist, kann erreicht werden, dass der Metallstift 49 mit einer vorbestimmten Mindestanpresskraft gegen einen elektrischen Kontakt der Energiespeichereinrichtung 46 gedrückt wird. Zum Schließen eines Stromkreises können der mit Stutzen 48 und das Rohr der es Ohrsteckers 33 aus einem elektrisch leitenden Material, insbesondere Metall, gebildet sein.

Anstelle oder zusätzlich zu der Aufteilung in die Kammern 37, 38 mit der Trennwand 39 kann vorgesehen sein, dass die Einrichtung 40 und die Mikrofoneinrichtung 41 abwechselnd betrieben werden und hierzu eine elektronische Wippe oder Wippschaltung vorgesehen ist, die zwischen den beiden Einrichtungen 40, 41 abwechselnd umschaltet.

Durch die Schaltungsplatine 42 kann eine Steuerung mittels der Steuerschaltung 44 vorgesehen sein, welche die Lautsprechereinrichtung 40 und die Mikrofonvorrichtung 41 abschaltet, sodass kein Stromfluss entsteht, wenn keine Telefonverbindung aufgebaut ist. Bevorzugt ist auch vorgesehen, die Kommunikationsverbindung 24 abzuschalten. Das Einschalten des Ohrstücks 17 kann über das Bedienelement 45 erfolgen und/oder durch ein Aufwecksignal 53 des Endgeräts 14, durch welches die Kommunikationseinheit 43 dazu veranlasst werden kann, die Kommunikationsverbindung 24 mit dem Endgerät 14 aufzubauen.

Durch den Ohrschmuck 16 ergeben sich erhebliche Vorteile in Bezug auf die Funktion, das Design und die Benutzerfreundlichkeit. Durch die vollständige Integration der Funktionalitäten eines drahtlosen Kommunikationsgeräts in ein Schmuckstück oder einen Ohrschmuck 16 wird nicht nur eine natürliche, gerichtete Schallübertragung von dem Ohrläppchen oder der Ohrmuschel 18 hin zum Gehörgang 20 erreicht, sondern es lässt sich auch problemlos eine Rückkopplung zwischen der Lautsprechereinrichtung 40 und der Mikrofoneinrichtung 41 vermeiden. Die Verteilung des Gewichts erfolgt vor und hinter dem Ohr 12 durch Austarieren des Gewichts des Schmuckkörpers 23 und des Abschlusselements 36. Hierdurch wird der Komfort beim Tragen des Ohrstücks 17 gewährleistet, während gleichzeitig die Kommunikation oder ein Telefongespräch diskret ist. Das Design ermöglicht es insbesondere, das Ohrstück permanent zu tragen. Um Telefongespräche abzuwickeln ist es nicht mehr erforderlich, das Endgerät 14 aus z.B. einer Tasche zu nehmen.

Indem das Ohrstück 17 abgeschaltet bleibt, solange es nicht in Benutzung ist, wird sowohl Batterieenergie eingespart als auch eine Strahlungsbelastung aufgrund der Kommunikationsverbindung 24 reduziert. Eingehende Telefongespräche können dennoch sofort angenommen werden, ohne dass hierzu das Endgerät 14 selbst bedient oder berührt werden muss. Es kann somit z.B. bequem in der Handtasche verbleiben und muss nicht extra gesucht werden.

Die Kommunikationsverbindung 24 ermöglicht es auch, als Endgerät 14 zum Beispiel einen Tablet-PC oder einen Personal-Computer (PC) zu nutzen.

Zusätzlich oder alternativ zur Übertragung eines Telefongesprächs kann der Ohrschmuck 16 auch als Audiogerät genutzt werden.

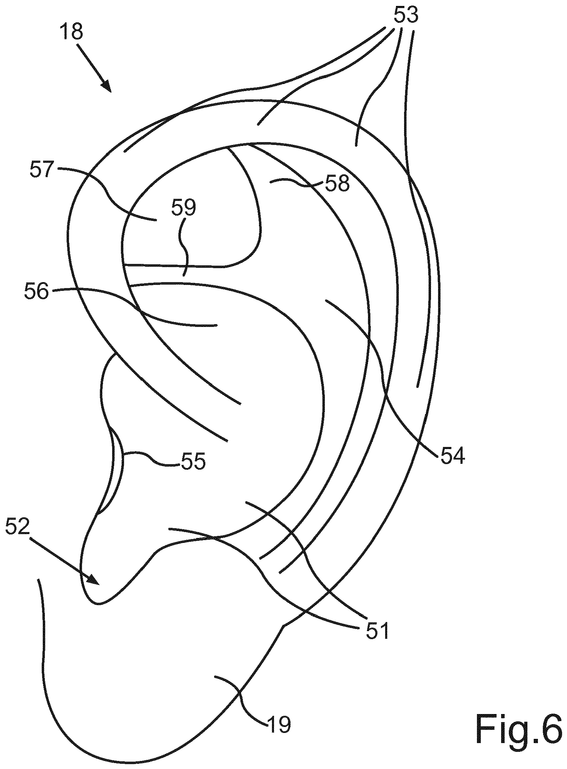

Fig. 6 zeigt die Ohrmuschel 18 ohne den Ohrschmuck 16. Die Ohrmuschel 18 ist das Außenohr des Benutzers 10. Dargestellt sind die folgenden Elemente:
- 19: Ohrläppchen
- 51: Cavum Conchae Inferior
- 52: Icisura Intertragica
- 53: Helix
- 54: Antihelix
- 55: Tragus
- 56: Cavum Conchae Superior
- 57: Fossa Triangularis
- 58: Obere Antihelixwurzel
- 59: Untere Antihelixwurzel

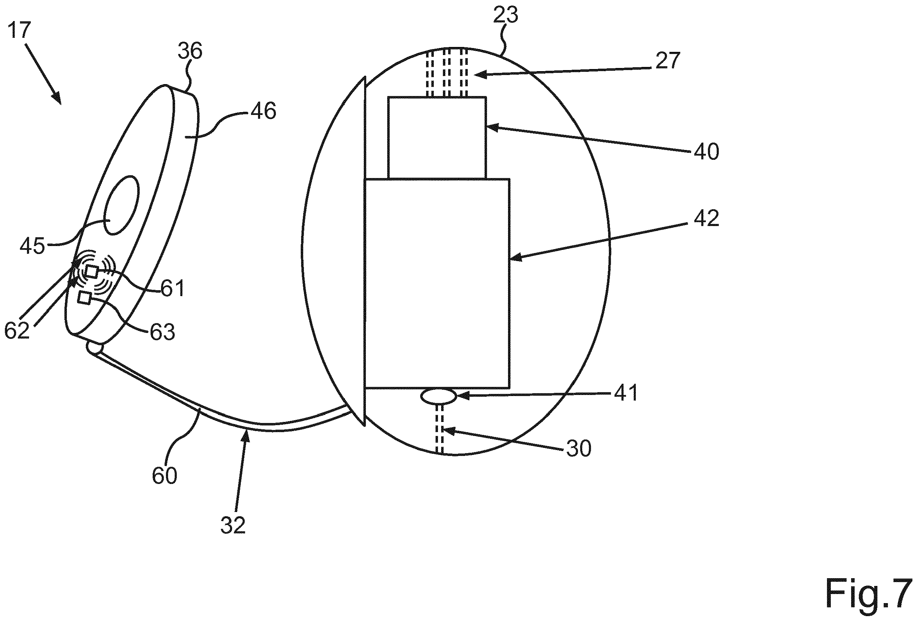

Fig. 7 zeigt ein Ohrstück 17, bei welchem ein Bedienelement 45, beispielsweise ein Schalter, in der Halteeinrichtung 32 bereitgestellt ist, beispielsweise an dem Abschlusselement. Die in Fig. 7 gezeigte Ausführungsform hat eine Halteeinrichtung 32, die als so genannter Clip ausgestaltet ist, also keinen Ohrstecker benötigt. Das Bedienelement 45 kann bei einer Bedienung beispielsweise eine elektrische Spannung modulieren, die durch ein Kabel oder allgemein einem elektrischen Leitelement in einem Verbindungsstück 60 übertragen wird, nämlich von der Energiespeichereinrichtung 46 im Abschlusselement 36 zu der Schaltungsplatine 42 im Schmuckkörper 23.

Des Weiteren kann unabhängig davon in jeder Ausführungsform ein Aktuator oder Vibrationselement 61 bereitgestellt sein, welches ein Vibrationssignal 62 für eine Körperschallübertragung in den Kopf 11 des Benutzers 10 erzeugen kann. Das Vibrationselement 61 kann beispielsweise in Abhängigkeit von einem Sensorsignal eines Sensors 63 aktiviert werden. Der Sensor 63 kann beispielsweise ein Näherungssensor oder ein Drucksensor oder ein Photosensors sein.

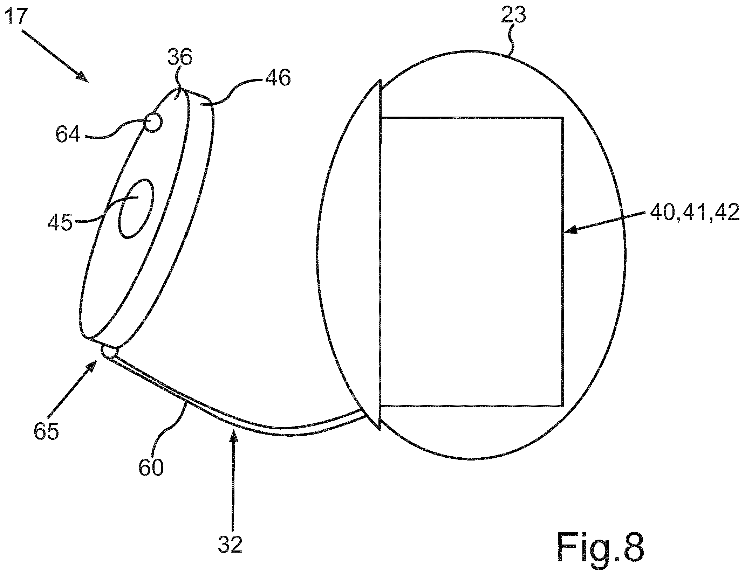

Fig. 8 zeigt ein Ohrstück 17, bei welchem ein Ladekontakt 64 für die Energiespeichereinrichtung 46 in dem Abschlusselement 36 bereitgestellt ist. Mittels eines Gelenks 65 kann das Abschlussstück 36 bezüglich des Verbindungselements 60 klappbar ausgestaltet sein, sodass das Abschlusselement 36 nicht nur zum Anclipsen des Ohrrings, sondern z.B. auch zum Einführen in ein Ladegerät ausgeklappt oder umgeklappt werden kann.

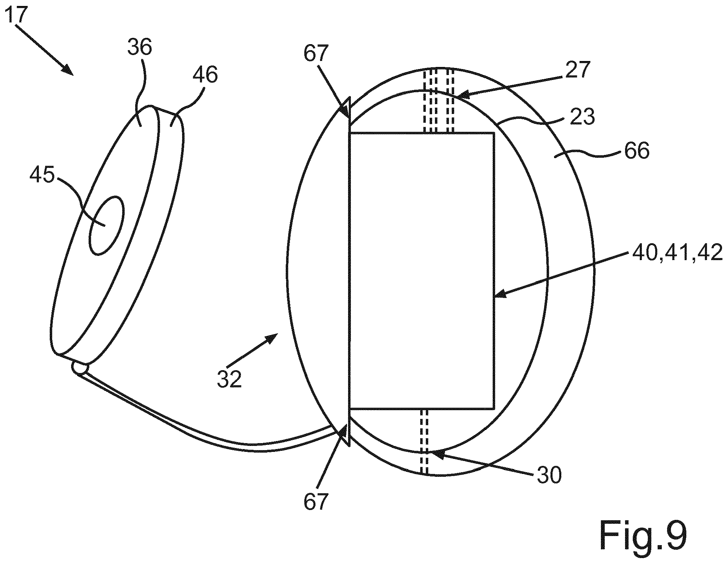

Fig. 9 veranschaulicht ein Ohrstück 17, bei welchem der Schmuckkörper 23 ein abnehmbares oder austauschbares Zierelement 66 aufweist. Die Austrittsöffnungen 27 für den Lautsprecherschall und die Eintrittsöffnung 30 für Mikrofoneinrichtung können auch in den Zierelement 66 bereitgestellt sein. Das Zierelement 66 kann beispielsweise als eine Schmuckkappe ausgestaltet sein. Mittels eines Befestigungsmechanismus 67 kann das Zierelement 66 beispielsweise an der Halteeinrichtung 32 gehalten sein. Der Befestigungsmechanismus kann beispielsweise ein Schraubmechanismus oder ein Schnappmechanismus sein.

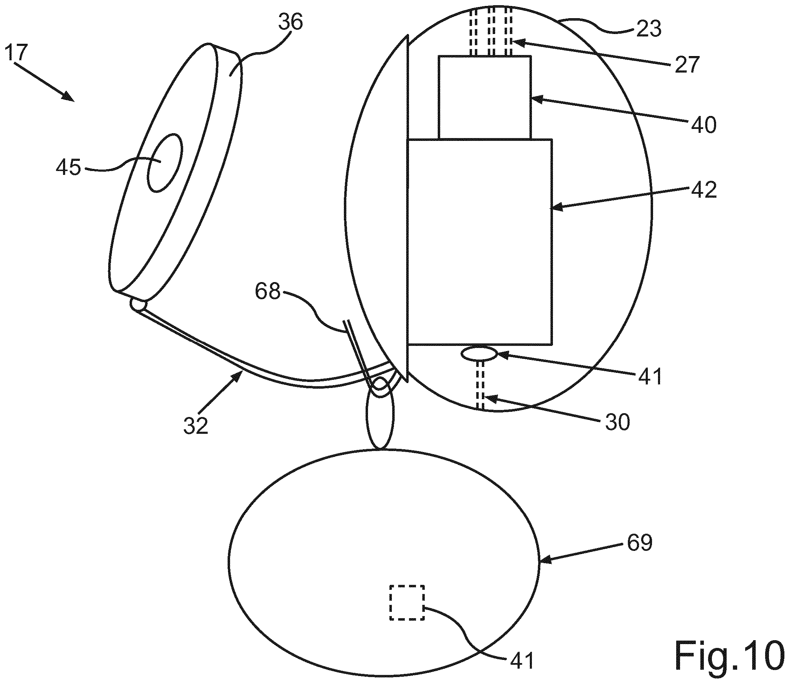

Fig. 10 zeigt ein Ohrstück 17, bei welchem ein zusätzliches Halteelement 68 bereitgestellt ist. Das Halteelement 68 kann beispielsweise als ein Haken ausgestaltet sein. An dem Halteelement 68 kann ein austauschbares Zusatz Zierelement 69 befestigt sein. Beispielsweise kann als Zusatzzierelement 69 ein Anhänger an das Halteelement 68 angehängt sein. In dem Zusatz Zierelement 69 kann beispielsweise auch die Mikrofoneinrichtung 41 (gestrichelt dargestellt) integriert sein. Hierdurch kann ein Abstand zwischen der Lautsprechereinrichtung 40 und der Mikrofoneinrichtung 41 vergrößert werden.

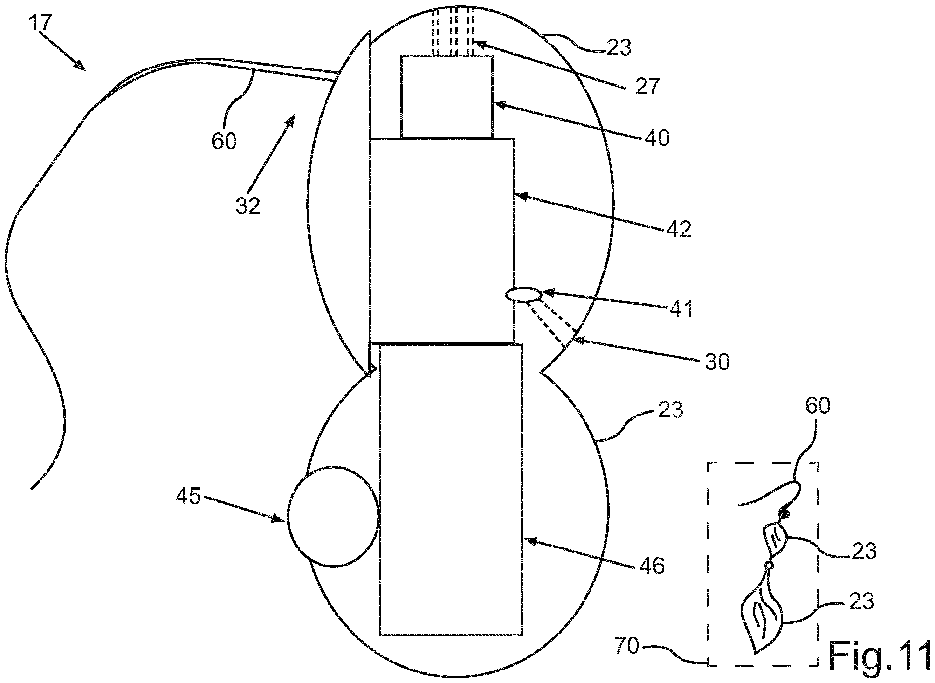

Fig. 11 zeigt ein Ohrstück 17, bei welchem die Energiespeichereinrichtung 46 in den Schmuckkörper 23 integriert sein kann. Eine perspektivische Ansicht 70 veranschaulicht eine mögliche Form des Schmuckkörpers 23.

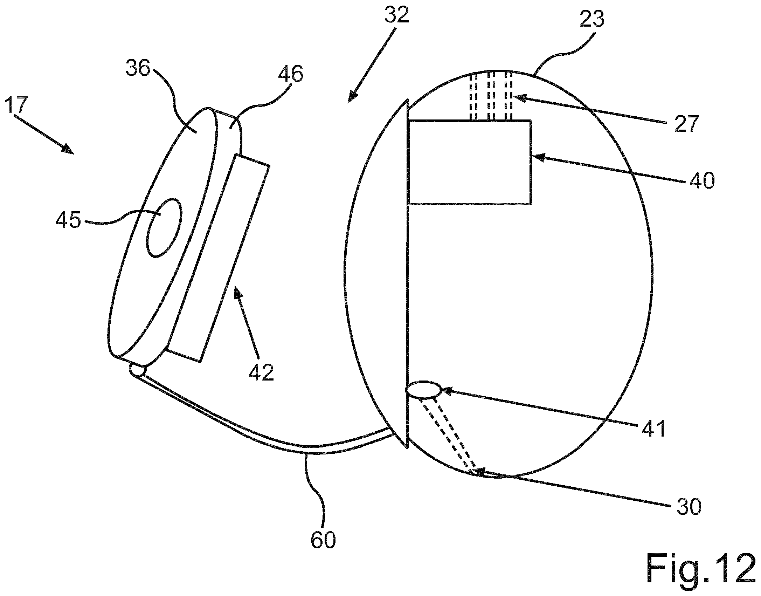

Fig. 12 zeigt ein Ohrstück 17, bei welchem die Schaltungsplatine 42 mit dem Bluetooth-Funkmodul, einer Antenne und möglichen Sensoren in dem Abschlussstück 36 zusammen mit einer Energiespeichereinrichtung 46 integriert ist.

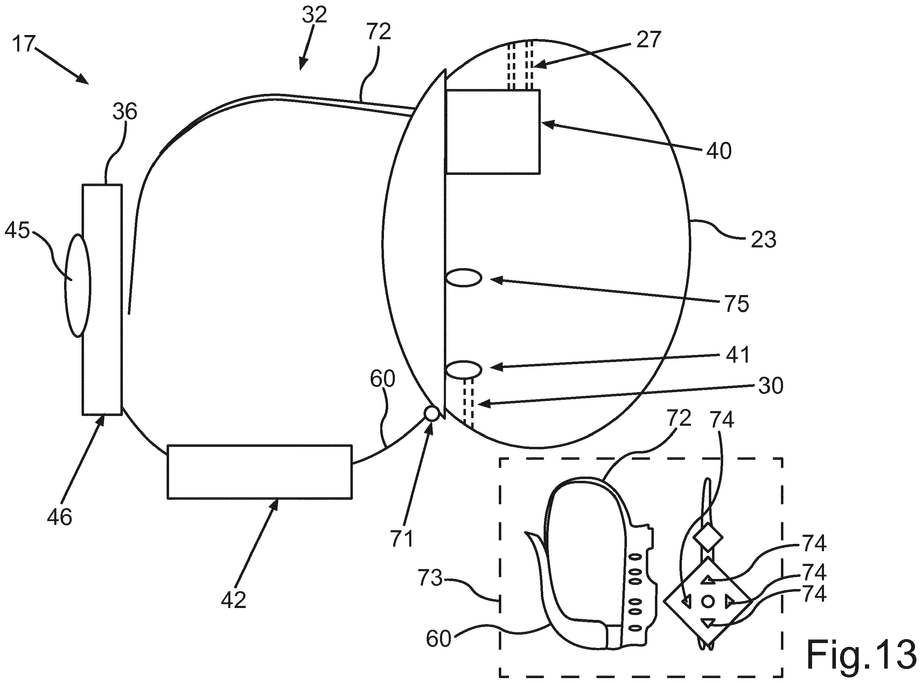

Fig. 13 zeigt ein Ohrstück 17, bei welchem die Schaltungsplatine 42 mit dem Bluetooth-Funkmodul in den Verbindungsstück 60 der Halteeinrichtung 32 zwischen dem Schmuckkörper 23 und dem Abschlusselement 36 mit der Energiespeichereinrichtung 46 angeordnet ist. Mittels eines Scharnier 71 kann das Verbindungsstück 60 bezüglich des Schmuckkörpers 23 verschwenkbar angeordnet sein. Ein Gegenstück 72 zum Verbindungsstück 60 kann als Ohrstecker für das Ohrloch 34 vorgesehen sein.

In Fig. 13 sind auch eine Seitenansicht und eine Frontansicht 73 einer möglichen Form des Verbindungsstücks 60 dargestellt. Die Anordnung der Schaltungsplatine 42 im Abschlusselement 36 (siehe Fig. 12) oder im Verbindungsstück 60 (siehe Fig. 13) weist den Vorteil auf, dass der Schmuckkörper 23 beispielsweise auch Durchgangsöffnungen 74 aufweisen kann.

Die Fig. 12 und Fig. 13 dargestellten Ausführungsformen gehen vor, dass die Schaltungsplatine 42 nicht im Schmuckkörper 23 angeordnet ist. Falls auf der Schaltungsplatine 42 zumindest ein Sensor zum Erfassen einer Bedienen Geste oder Bedienhandlung vorgesehen ist, kann der zumindest eine Sensor bei diesen Ausführungsformen als Sensor 75 in den Schmuckkörper 23 integriert sein. Hierdurch bleibt der zumindest eine Sensor von der Umgebung 21 aus erreichbar oder annäherbar.

Insgesamt zeigen die Beispiele, wie durch die Erfindung ein am Ohr tragbares Schmuckstück unauffällig um die Funktion eines sogenannten Headsets mit Lautsprecher und Mikrofon erweitert werden kann.

## Patentansprüche

1. Ohrschmuck (16) aufweisend zumindest ein an jeweils einem Ohr (12) eines Benutzers (10) zu tragendes Ohrstück (17), wobei jedes Ohrstück (17) jeweils aufweist:
- einen in einer vorbestimmten Tragestellung (T) an einer vom Kopf (11) des Benutzers (10) abgewandten Seite (22) des Ohrs (12) zu tragenden, von einer Umgebung (21) aus sichtbaren Schmuckkörper (23) und
- eine Halteeinrichtung (32) zum Halten des Schmuckkörpers (23) in der Tragestellung (T) an einer Ohrmuschel (18), insbesondere an einem Ohrläppchen (19) oder an einer Helix des Ohres (12), wobei in der Tragestellung (T) der Gehörgang (20) des Ohres (12) frei bleibt,
wobei in dem zumindest einen Ohrstück (17) insgesamt folgende elektrische Komponenten (40, 41, 43) vollständig, in der Tragestellung von außen unsichtbar integriert sind:
- eine Mikrofoneinrichtung (41) zum Erfassen eines von einem Mund (13) des Kopfes (11) zu dem Ohrstück (17) gelangten Stimmschalls (29),
- eine Lautsprechereinrichtung (40) zum Abstrahlen eines Lautsprecherschalls (26) aus dem jeweiligen Ohrstück (17) in Richtung des Gehörganges (20) des jeweiligen Ohres (12), und
- eine Kommunikationseinheit (43) zum Bereitstellen einer bidirektionalen, kabellosen Kommunikationsverbindung (24) zu einem portablen Endgerät (14) des Benutzers (10), wobei die Kommunikationseinheit (43) dazu eingerichtet ist, über die Kommunikationsverbindung (24) ein Mikrofonsignal (31) der Mikrofoneinrichtung (41) an das Endgerät (14) auszusenden und aus dem Endgerät (14) ein Lautsprechersignal (25) für die Lautsprechereinrichtung (40) zu empfangen, **dadurch gekennzeichnet, dass**
die Mikrofoneinrichtung (41) und die Lautsprechereinrichtung (40) in dem Schmuckkörper (23) eines gemeinsamen Ohrstücks (17) integriert sind und der Schmuckkörper (23) als ein Gehäuse (23') ausgestaltet ist, welches zumindest eine in der Tragestellung (T) dem Gehörgang (20) zugewandte Austrittsöffnung (27) für den Lautsprecherschall (26) und an einer gegenüberliegenden Seite eine Eintrittsöffnung (30) für den Stimmschall (29) aufweist und dass
die Lautsprechereinrichtung (40) über eine Richteinheit (27, 27') mit der Umgebung (21) gekoppelt ist, wobei die Richteinheit (27, 27') dazu eingerichtet ist, den Lautsprecherschall (26) in der Tragestellung (T) in eine Richtung zu dem Gehörgang (20) hin auszurichten, so dass der Lautsprecherschall (26) nach dem Austritt aus dem Ohrstück (17) in der Umgebung (21) eine Hauptausbreitungsrichtung (28) aufweist, entlang welcher der Lautsprecherschall (26) in den Gehörgang (20) gelangt oder diesen zumindest passiert, wobei
durch die Anordnung an der Ohrmuschel (18) und das Abstrahlen des Lautsprecherschalls (26) in Richtung des Gehörganges (20) eine akustische Kopplung zwischen einer schwingungsfähigen Luftsäule im Gehörgang (20) und der Lautsprechereinrichtung (40) erreicht ist.

2. Ohrschmuck (16) nach Anspruch 1, wobei in der Tragestellung zusätzlich zum Gehörgang (20) auch die Incisura Intertragica (52) und der Cavum Conchae Inferior (51) frei bleiben.

3. Ohrschmuck (16) nach einem der vorhergehenden Ansprüche, wobei die Richteinheit (27, 27') einen in dem Ohrstück (17) angeordneten Trichter und/oder ein in dem Ohrstück (17) angeordnetes Rohr (27') und/oder mehrere in einer Außenwandung des Ohrstücks (16) angeordnete Durchgangsöffnungen (27) vorsieht.

4. Ohrschmuck (16) nach Anspruch 3, wobei für eine Rückkopplungsunterdrückung eines Schallanteils des Lautsprecherschalls (26), der aus der Lautsprechereinrichtung (40) kommt und sich in Richtung zur Mikrofoneinrichtung (41) ausbreitet,
a) das Gehäuse (23') zwei Kammern (37, 38) aufweist und die Mikrofoneinrichtung (41) und die Lautsprechereinrichtung (40) in unterschiedlichen der Kammern (37, 38) angeordnet sind, und/oder
b) eine Prozessoreinheit des Ohrschmucks dazu eingerichtet ist, ein von der Mikrofoneinrichtung (41) erfasstes Schallsignal des Schallanteils zu dämpfen.

5. Ohrschmuck (16) nach einem der vorhergehenden Ansprüche, wobei das zumindest eine Ohrstück insgesamt zumindest ein Bedienelement (45) für eine Lautstärkeregulierung zum Einstellen einer Lautstärke des Lautsprecherschalls (26) und/oder einer Verstärkung des Mikrofonsignals (31) in Abhängigkeit von zumindest einem Bediensignal aufweist, wobei das zumindest eine Bedienelement (45) umfasst:
zumindest einen Sensor, der ein Bediensignal in Abhängigkeit von einer Streichbewegung eines Objekts an einer Außenoberfläche des zumindest einen Ohrstücks erzeugt; und/oder
- einen Drehlagesensor der bei Drehen eines Teilstücks des Ohrschmucks ein Bediensignal in Abhängigkeit von einer absoluten Drehlage im Raum oder in Abhängigkeit von einer relativen Drehlage des Teilstück bezüglich eines Rests des Ohrschmucks erzeugt.

6. Ohrschmuck (16) nach einem der vorhergehenden Ansprüche, wobei das zumindest eine Ohrstück insgesamt zumindest ein Bedienelement (45) für eine Lautstärkeregulierung zum Einstellen einer Lautstärke des Lautsprecherschalls (26) und/oder einer Verstärkung des Mikrofonsignals (31) in Abhängigkeit von zumindest einem Bediensignal aufweist, wobei das zumindest eine Bedienelement (45) umfasst:
- einen Sensor, der ein Bediensignal in Abhängigkeit von einer Erschütterung des zumindest eines Ohrstücks (17) erzeugt.

7. Ohrschmuck (16) nach einem der vorhergehenden Ansprüche, wobei das zumindest eine Ohrstück insgesamt zumindest ein Bedienelement (45) für eine Lautstärkeregulierung zum Einstellen einer Lautstärke des Lautsprecherschalls (26) und/oder einer Verstärkung des Mikrofonsignals (31) in Abhängigkeit von zumindest einem Bediensignal aufweist, wobei das zumindest eine Bedienelement (45) umfasst:
- eine Analyseeinrichtung, die in dem Mikrofonsignal (31) der Mikrofoneinrichtung (41) vorbestimmte Impulse detektiert und daraufhin ein Bediensignal erzeugt.

8. Ohrschmuck (16) nach einem der vorhergehenden Ansprüche, wobei eine Energiespeichereinrichtung (46) zum Betreiben der elektrischen Komponenten (40, 41, 43) in den Ohrschmuck (16) derart integriert ist, dass die Energiespeichereinrichtung (46) in der Tragestellung hinter der Ohrmuschel (12) zwischen Ohrmuschel (12) und Kopf (11) des Benutzers (10) oder unter der Ohrmuschel (12) angeordnet ist, wobei die Energiespeichereinrichtung (46) über zumindest ein elektrisches Leitelement elektrisch mit den elektrischen Komponenten (40, 41, 43) verbunden ist und
eine Modulationseinrichtung dazu eingerichtet ist, zumindest ein Sensorsignal und/oder zumindest ein Signal eines Bedienelements auf eine in dem zumindest einen elektrischen Leitelement bereitgestellte elektrische Spannung aufzumodulieren.

9. Ohrschmuck (16) nach einem der vorhergehenden Ansprüche, wobei zumindest ein Sensor in dem Ohrschmuck bereitgestellt ist, der dazu ausgelegt ist, zumindest eine vorbestimmte Bewegung des Kopfes (11) im Raum zu erfassen und dann ein vorbestimmtes Steuersignal für die elektrischen Komponenten (40, 41, 43) zu erzeugen.

10. Ohrschmuck (16) nach einem der vorhergehenden Ansprüche, wobei eine Steuerschaltung (44) des Ohrstücks (17) dazu eingerichtet ist, in einem Ruhezustand die Lautsprechereinrichtung (40) und die Mikrofoneinrichtung (41) stromlos zu halten und die Steuerschaltung (44) dazu eingerichtet ist, in Abhängigkeit von einem kabellos empfangenen Aufwecksignal des Endgeräts (14) die Lautsprechereinrichtung (40) und die Mikrofoneinrichtung (41) mit Strom zu versorgen.

11. Ohrschmuck (16) nach einem der vorhergehenden Ansprüche, wobei der Schmuckkörper (23) zumindest eines Ohrstücks (17) jeweils an einer in der Tragestellung (T) der Umgebung (21) zugewandten Seite ein von außen sichtbares, austauschbares Zierelement (23') aufweist, welches von einem Rest des Schmuckkörpers (23) zerstörungsfrei und reversibel lösbar ausgestaltet ist, und/oder mindestens ein Halteelement zum wahlweise Befestigen und/oder Anhängen zumindest eines austauschbaren Zusatzzierelements aufweist.

12. Ohrschmuck (16) nach einem der vorhergehenden Ansprüche, der Ohrschmuck zumindest einen Sensor zur Erfassung von Körperdaten, insbesondere Herzfrequenz und Temperatur, aufweist.

13. Ohrschmuck (16) nach einer der vorhergehenden Ansprüche, der Ohrschmuck über ein Panik-Bedienelement verfügt, das bei Betätigung einen Notruf auslöst.

14. Ohrschmuck (16) nach einem der vorhergehenden Ansprüche, der Ohrschmuck über eine Haltefunktion verfügt, die dazu eingerichtet ist, in Abhängigkeit von einer vorbestimmten ersten Bediengeste eingehende Sprechverbindungen halten und in Abhängigkeit von einer vorbestimmten zweiten Bediengeste die Sprechverbindung wieder durchzuschalten.

15. Ohrschmuck (16) nach einem der vorhergehenden Ansprüche, wobei ein Vibrationselement bereitgestellt ist, welches dazu eingerichtet ist, in Abhängigkeit von einem Aktivierungssignal des Endgeräts (14) bei Eintreffen einer Nachricht oder Anrufes ein Vibrationssignal zu erzeugen und/oder bei Andrücken des Ohrstücks (17), in welchem das Vibrationselement angeordnet ist, an den Kopf (11) das Lautsprechersignal (25) als einen Körperschall in den Kopf (11) des Benutzers (10) abzugeben.

## Claims

1. Ear jewelry (16) comprising at least one earpiece (17) each to be worn on one ear (12) of a user (10), while each earpiece (17) comprises:
- a jewelry body (23) that is to be worn in a predetermined wearing position (T) on a side (22) of the ear (12) facing away from the head (11) of the user (10), such that it is visible to an environment (21) and
- a holding device (32) for holding the jewelry body (23) in the wearing position (T) on an auricle (18), in particular on an earlobe (19) or on a helix of the ear (12), wherein in the wearing position (T) the auditory canal (20) of the ear (12) remains unobstructed, wherein
in the at least one earpiece (17) the following electrical components (40, 41, 43) are completely integrated and invisible from the outside while in the wearing position:
- a microphone device (41) for detecting a voice sound (29) which passes from a mouth (13) of the head (11) to the earpiece (17),
- a loudspeaker device (40) for emitting a loudspeaker sound (26) from the respective earpiece (17) in the direction of the auditory canal (20) of the respective ear (12), and
- a communication unit (43) for providing a bidirectional, wireless communication link (24) to a portable device (14) of the user (10), wherein the communication unit (43) is adapted to send a microphone signal (31) of the microphone device (41) to the device (14) and to receive a loudspeaker signal (25) from the device (14) for the loudspeaker device (40) over the communication link (24),
**characterized in that**
the microphone device (41) and the loudspeaker device (40) are integrated into the jewelry body (23) of a common earpiece (17) and the jewelry body (23) is designed as a housing (23'), which provides for the loudspeaker sound (26) at least one outlet opening (27) that, in the wearing position (T), is facing the auditory canal (20) and at an opposite side an entrance opening (30) for the vocal sound (29) and that
the loudspeaker device (40) is coupled to the environment (21) via a directivity unit (27, 27'), wherein, in the wearing position (T), the directivity unit (27, 27') is adapted to direct the loudspeaker sound (26) into a direction towards the auditory canal (20), so that after exiting the earpiece (17) into the environment (21) the loudspeaker sound (26) has a main propagation direction (28) along which the loudspeaker sound (26) reaches the ear canal (20) or at least passes by the ear canal (20), wherein due to the arrangement on the auricle (18) and the emission of the loudspeaker sound (26) in the direction of the auditory canal (20), an acoustic coupling between an oscillatory air column in the auditory canal (20) and the loudspeaker device (40) is achieved.

2. Ear jewelry (16) according to claim 1, wherein in addition to the auditory canal (20) the incisura intertragica (52) and the cavum conchae inferior (51) also remain free in the wearing position.

3. Ear jewelry (16) according to any of the preceding claims, wherein the directivity unit (27, 27') comprises a funnel arranged in the earpiece (17) and/or a pipe (27') arranged in the earpiece (17) and/or several through-holes (27) which are arranged in an outer wall of the earpiece (16).

4. An ear jewelry (16) according to claim 3, wherein for feedback suppression of a sound portion of the loudspeaker sound (26) which is coming from the loudspeaker device (40) and propagating towards the microphone means (41),
a) the housing (23') has two chambers (37, 38) and the microphone device (41) and the loudspeaker device (40) are arranged in different ones of the chambers (37, 38), and/or
b) a processor unit of the ear jewelry (16) is adapted to attenuate a sound signal of the sound portion detected by the microphone device (41).

5. Ear jewelry (16) according to any of the preceding claims, wherein the at least one earpiece (17) as a whole includes at least one operating element (45) for a volume control for adjusting a volume of the loudspeaker sound (26) and/or amplification of the microphone signal (31) in dependence on at least an operating signal, wherein the at least one operating element (45) comprises:
- at least one sensor that generates the operating signal in response to a sweeping movement of an object on an outer surface of the at least one earpiece; and/or
- a rotational position sensor which generates upon turning of a portion of the ear jewelry (16) the operating signal in response to an absolute rotational position in space or in dependence on a relative rotational position of the portion with respect to a remainder of the ear jewelry (16).

6. Ear jewelry (16) according to any of the preceding claims, wherein the at least one earpiece (17) as a whole includes at least one operating element (45) for a volume control for adjusting a volume of the loudspeaker sound (26) and/or amplification of the microphone signal (31) in dependence on at least an operating signal, wherein the at least one operating element (45) comprises:
sensor that generates the operating signal as a function of a shaking of the at least one earpiece (17).

7. Ear jewelry (16) according to any of the preceding claims, wherein the at least one earpiece (17) as a whole includes at least one operating element (45) for a volume control for adjusting a volume of the loudspeaker sound (26) and/or amplification of the microphone signal (31) in dependence on at least an operating signal, wherein the at least one operating element (45) comprises:
an analysis device which detects at least one predetermined pulse in the microphone signal (31) of the microphone device (41) and subsequently generates the operating signal.

8. Ear jewelry (16) according to any of the preceding claims, wherein an energy storage device (46) for operating the electrical components (40, 41, 43) is integrated into the ear jewelry (16) such that, in the wearing position, the energy storage device (46) is arranged either behind the auricle (12) between the auricle (12) and the head (11) of the user (10) or under the auricle (12), wherein the energy storage device (46) is electrically connected to the electrical components (40, 41, 43) via at least one electrical conducting element (43) and a modulation device is designed to modulate at least one sensor signal and/or at least one signal of an operating element on an electrical voltage provided in the at least one electrical conducting element (43).

9. Ear jewelry (16) according to any of the preceding claims, wherein at least one sensor is provided in the ear jewelry (16) which is designed to detect at least one predetermined movement of the head (11) in space and then generates a predetermined control signal for the electrical components (40, 41, 43).

10. Ear jewelry (16) according to any of the preceding claims, wherein a control circuit (44) of the earpiece (17) is adapted to keep the loudspeaker device (40) and the microphone device (41) de-energized in a idle state and the control circuit (44) is configured to supply power to the loudspeaker device (40) and the microphone device (41) as a function of a wirelessly received wake-up signal of the device (14).

11. Ear jewelry (16) according to any of the preceding claims, wherein the jewelry body (23) of at least one earpiece (17) includes an externally visible, exchangeable decorative element (23'), which is designed to be non-destructively and reversibly detachable from a remainder of the jewelry body (23), and/or includes at least one holding element for selectively attaching and/or hanging at least one interchangeable additional decorative element which is facing the environment (21) in the wearing position (T).

12. Ear jewelry (16) according to any of the preceding claims, wherein the ear jewelry comprises at least one sensor for detecting body data, in particular a heart rate and a temperature.

13. Ear jewelry (16) according to any of the preceding claims, wherein the ear jewelry (16) includes a panic control element, which triggers an emergency call when actuated.

14. Ear jewelry (16) according to any of the preceding claims, wherein the ear jewelry comprises a holding function, which is adapted to hold, in response to a predetermined first operating gesture, incoming speech connections and turn on the speech connection again in response to a predetermined second operating gesture.

15. Ear jewelry (16) according to any of the preceding claims, wherein a vibration element is provided which is adapted to generate a vibration signal in response to an activation signal of the device (14) upon arrival of a message or call and/or to generate a vibration signal upon pressing the earpiece (17), in which the vibrating element is arranged, to the head (11) of the user (10) in order to deliver the loudspeaker signal (25) as a structure-borne sound into the head (11).

## Revendications

1. Boucle d'oreille (16) comprenant au moins un écouteur (17) destiné à être porté sur une oreille respective (12) d'un utilisateur (10), chaque écouteur (17) comprenant :
- un corps de boucle d'oreille (23) destiné à être porté dans une position de portage prédéterminée (T) sur un côté (22) de l'oreille (12) opposé à la tête de l'utilisateur (10) et étant visible depuis les environs, et
- un dispositif de maintien (32) permettant de maintenir le corps de boucle d'oreille (23) dans la position de portage (T) à un pavillon de l'oreille (18), surtout à un lobe de l'oreille (19) ou à une hélix de l'oreille (12), dans lequel le conduit auditif (20) de l'oreille (12) reste libre dans la position de portage (T),
dans lequel dans l'au moins un écouteur (17) dans son ensemble les composants électriques suivants (40, 41, 43) sont intégrés complètement et de manière invisible de l'extérieur dans la position de portage:
- un dispositif formant microphone (41) pour détecter un son de voix (29) allant de la bouche (13) de la tête (11) à l'écouteur (17),
- un dispositif de haut-parleur (40) pour émettre un son du haut-parleur (26) venant de l'écouteur (17) respectif en direction du conduit auditif (20) de l'oreille respective (12), et
- une unité de communication (43) destinée à fournir une liaison de communication bidirectionnelle sans fil (24) avec un terminal portable (14) de l'utilisateur (10), dans laquelle l'unité de communication (43) est adaptée pour transmettre sur la liaison de communication (24) un signal de microphone (31) du dispositif formant microphone (41) au terminal (14) et pour recevoir à partir du terminal (14) un signal de haut-parleur (25) pour le dispositif de haut-parleur (40),
**caractérisé en ce que**
le dispositif formant microphone (41) et le dispositif de haut-parleur (40) sont intégrés dans le corps de boucle d'oreille (23) d'un écouteur (17) commun et le corps de boucle d'oreille (23) est conçu sous forme de boîtier (23') qui comporte au moins une ouverture de sortie (27) pour le son du haut-parleur (26) tournée vers le conduit auditif (20) dans la position de portage (T) et sur un côté opposé comporte une ouverture d'entrée (30) pour le son de voix (29)
et que
le dispositif de haut-parleur (40) est couplé par une unité de pointage (27, 27') avec les environs, dans lequel l'unité de pointage (27, 27') est adaptée pour diriger le son du haut-parleur (26) dans la position de portage (T) en une direction vers le conduit auditif (20) de sorte que le son du haut-parleur (26) après la sortie de l'écouteur (17) dans les environs (21) comporte une direction de propagation principale (28) le long de laquelle le son du haut-parleur (26) arrive dans le conduit auditif (20) ou au moins le son du haut-parleur (26) passe le conduit auditif (20),
dans lequel
par l'agencement au pavillon de l'oreille (18) et l'émission du son du haut-parleur (26) en direction du conduit auditif (20) un couplage acoustique est obtenu entre une colonne d'air vibrante dans le conduit auditif (20) et le dispositif de haut-parleur (40).

2. Boucle d'oreille (16) selon la revendication 1, dans laquelle dans la position de portage en plus du conduit auditif (20) aussi *l'incisura intertragica* (52) et le *cavum chonchae inferior* (51) restent libres.

3. Boucle d'oreille (16) selon l'une quelconque des revendications précédentes, dans laquelle l'unité de pointage (27, 27') prévoit un entonnoir agencé dans l'écouteur (17) et/ou un tube (27') agencé dans l'écouteur (17) et/ou plusieurs ouvertures de passage (27) agencées dans une paroi extérieure de l'écouteur (16).

4. Boucle d'oreille (16) selon la revendication 3, dans laquelle pour une suppression des rétroactions acoustiques d'une fraction sonore du son du haut-parleur (26) venant du dispositif de haut-parleur (40) et se propageant en direction vers le dispositif formant microphone (41)
a) le boîtier (23') comporte deux chambres (37, 38) et le dispositif formant microphone (41) et le dispositif de haut-parleur (40) sont agencés dans différentes des chambres (37, 38), et/ou
b) une unité processeur de la boucle d'oreille est adaptée pour atténuer un signal sonore détecté par le dispositif formant microphone (41).

5. Boucle d'oreille (16) selon l'une quelconque des revendications précédentes, dans laquelle l'au moins un écouteur comporte dans son ensemble au moins un élément de commande (45) pour un réglage de volume pour régler un volume du son du haut-parleur (26) et/ou d'une amplification du signal de microphone (31) en fonction d'au moins un signal de commande, dans lequel l'au moins un élément de commande (45) comporte:
au moins un capteur qui produit un signal de commande en fonction d'un mouvement d'effleurement d'un objet sur une surface extérieure de l'au moins un écouteur; et/ou
- un capteur de position de rotation qui lors de la rotation d'une section partielle de la boucle d'oreille produit un signal de commande en fonction d'une position de rotation absolue dans l'espace ou en fonction d'une position de rotation relative de la section partielle relatif à un reste de la boucle d'oreille.

6. Boucle d'oreille (16) selon l'une quelconque des revendications précédentes, dans laquelle l'au moins un écouteur dans son ensemble comporte au moins un élément de commande (45) pour un réglage de volume pour régler un volume du son du haut-parleur (26) et/ou d'une amplification du signal de microphone (31) en fonction d'au moins un signal de commande, dans lequel l'au moins un élément de commande (45) comporte:
- un capteur qui génère un signal de commande en fonction d'un ébranlement de l'au moins un écouteur (17).

7. Boucle d'oreille (16) selon l'une quelconque des revendications précédentes, dans laquelle l'au moins un écouteur dans son ensemble comporte au moins un élément de commande (45) pour un réglage de volume pour régler un volume du son du haut-parleur (26) et/ou d'une amplification du signal de microphone (31) en fonction d'au moins un signal de commande, dans lequel l'au moins un élément de commande (45) comporte:
- un moyen d'analyse qui détecte des impulsions prédéterminées dans le signal de microphone (31) du dispositif formant microphone (41) et ensuite produit un signal de commande.

8. Boucle d'oreille (16) selon l'une quelconque des revendications précédentes, dans laquelle un dispositif de stockage d'énergie (46) pour le fonctionnement des composants électriques (40, 41, 43) est intégrée dans la boucle d'oreille (16) de sorte que le dispositif de stockage d'énergie (46) dans la position de portage est agencé derrière le pavillon de l'oreille (12) entre le pavillon de l'oreille (12) et la tête (11) de l'utilisateur (10) ou sous le pavillon de l'oreille (12), dans lequel le dispositif de stockage d'énergie (46) est relié électriquement avec les composants électriques (40, 41, 43) par au moins un élément conducteur électrique et
- un moyen de modulation est adapté pour moduler au moins un signal de capteur et/ou au moins un signal de l'élément de commande sur une tension fournie dans l'au moins un élément conducteur électrique.

9. Boucle d'oreille (16) selon l'une quelconque des revendications précédentes, dans laquelle au moins un capteur est fourni dans la boucle d'oreille qui est adapté pour détecter au moins un mouvement prédéterminé de la tête (11) dans l'espace et pour produire ensuite un signal de commande prédéterminé pour les composants électriques (40, 41, 43).

10. Boucle d'oreille (16) selon l'une quelconque des revendications précédentes, dans laquelle un circuit de commande (44) de l'écouteur (17) est adaptée pour maintenir le dispositif de haut-parleur (40) et le dispositif formant microphone (41) sans courant dans un état de repos et le circuit de commande (44) est adaptée pour alimenter en courant le dispositif de haut-parleur (40) et le dispositif formant microphone (41) en fonction d'un signal de réveil du terminal (14) reçu sans fil.

11. Boucle d'oreille (16) selon l'une quelconque des revendications précédentes, dans laquelle le corps de boucle d'oreille (23) de l'au moins un écouteur (17) sur un côté respectif tourné vers les environs (21) dans la position de portage (T) comporte un élément décoratif (23') échangeable visible de l'extérieur qui est conçu de manière amovible sans dommage et réversiblement du reste du corps de boucle d'oreille (23) et/ou comporte au moins un élément de maintien pour une fixation ou accrochage sélectif de l'au moins un élément décoratif supplémentaire échangeable.

12. Boucle d'oreille (16) selon l'une quelconque des revendications précédentes, dans laquelle la boucle d'oreille (16) comporte au moins un capteur pour détecter des données corporelles, surtout la fréquence cardiaque et la température.

13. Boucle d'oreille (16) selon l'une quelconque des revendications précédentes, dans laquelle la boucle d'oreille (16) comporte un élément de commande d'alarme qui déclenche un appel d'urgence lorsqu'il est activé.

14. Boucle d'oreille (16) selon l'une quelconque des revendications précédentes, dans laquelle la boucle d'oreille (16) comporte une fonction d'attente qui, en fonction d'un premier geste de commande prédéterminé, est adaptée pour maintenir des communications vocales entrantes, et pour connecter de nouveau la communication vocale en fonction d'un second geste de commande prédéterminé.

15. Boucle d'oreille (16) selon l'une quelconque des revendications précédentes, dans laquelle un élément vibrant est fourni qui est adapté pour produire un signal de vibration en fonction d'un signal d'activation du terminal (14) lorsqu'un message ou un appel est reçu et/ou pour émettre le son du haut-parleur (26) comme un bruit du corps dans la tête (11) de l'utilisateur (10) lorsque l'écouteur (17), dans lequel l'élément vibrant est agencé, est appuyé à la tête (11).
